(19) Europäisches Patentamt / European Patent Office / Office européen des brevets

(11) **EP 4 401 833 B1**

(12) **EUROPEAN PATENT SPECIFICATION**


(45) Date of publication and mention of the grant of the patent:
**22.10.2025 Bulletin 2025/43**

(21) Application number: **22778038.4**

(22) Date of filing: **15.09.2022**

(51) International Patent Classification (IPC):
***A61P 25/00*** (2006.01) ***C07D 401/04*** (2006.01)
***C07F 9/09*** (2006.01) ***A61K 31/4439*** (2006.01)

(52) Cooperative Patent Classification (CPC):
**C07D 401/04; A61P 25/00; C07F 9/65583**

(86) International application number:
**PCT/GB2022/052328**

(87) International publication number:
**WO 2023/041909 (23.03.2023 Gazette 2023/12)**


(54) **GPR52 MODULATOR COMPOUNDS**

GPR52-MODULATORVERBINDUNGEN

COMPOSÉS MODULATEURS DE GPR52


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2021 GB 202113186**

(43) Date of publication of application:
**24.07.2024 Bulletin 2024/30**



(73) Proprietor: **Nxera Pharma UK Limited**
**Cambridge, Cambridgeshire CB21 6DG (GB)**

(72) Inventors:
- **WATSON, Stephen Paul**
  **Cambridge Cambridgeshire CB21 6DG (GB)**
- **SWAIN, Nigel Alan**
  **Cambridge Cambridgeshire CB21 6DG (GB)**
- **O'BRIEN, Michael Alistair**
  **Cambridge Cambridgeshire CB21 6DG (GB)**

(74) Representative: **Gill Jennings & Every LLP**
**The Broadgate Tower**
**20 Primrose Street**
**London EC2A 2ES (GB)**



(56) References cited:
**WO-A1-2016/176571 WO-A1-2019/053090**
**WO-A1-2021/090030**

- **NAKAHATA TAKASHI ET AL: "Design and synthesis of 1-(1-benzothiophen-7-yl)-1H-pyrazole, a novel series of G protein-coupled receptor 52 (GPR52) agonists", BIOORGANIC & MEDICINAL CHEMISTRY, vol. 26, no. 8, 1 May 2018 (2018-05-01), AMSTERDAM, NL, pages 1598 - 1608, XP055973257, ISSN: 0968-0896, DOI: 10.1016/j.bmc.2018.02.005**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 4 401 833 B1

## Description

This application relates to novel compounds and their use as prodrugs of G-protein coupled receptor 52 (GPR52) modulators. The compounds described herein may be useful in the treatment or prevention of diseases in which GPR52 receptors are involved or in which modulation of GPR52 receptors may be beneficial. The application is also directed to pharmaceutical compositions comprising these compounds and the manufacture and use of these compounds and compositions in the prevention or treatment of diseases in which GPR52 receptors are involved or in which modulation of GPR52 receptors may be beneficial.

## BACKGROUND OF THE INVENTION

G-protein coupled receptor 52 (GPR52) is a constitutively active Gs coupled orphan receptor which is highly expressed in the striatum and cortex. In the striatum GPR52 is expressed exclusively on dopamine D2 medium spiny neurons and in the cortex it is found on cortical pyramidal neurons expressing dopamine D1 receptors (Komatsu et al, 2014, PLoS One 9:e90134). Based on its localization and functional coupling, GPR52 is proposed to play a role in the modulation of fronto-striatal and limbic dopamine and may therefore have utility in the treatment of neuropsychiatric disorders. GPR52 agonists are thought to be particularly relevant to the treatment of schizophrenia, where they are hypothesized to improve cognition and negative symptoms indirectly by potentiating D1 signalling but alleviate positive symptoms through inhibition of D2-mediated signalling in the striatum.

GPR52 agonists could be used to treat psychiatric disorders related to dysfunction of the mesolimbic and mesocortical pathways. Examples include treatment of the positive, negative and cognitive symptoms of schizophrenia, depression, attention-deficit hyperactivity disorder, anxiety disorders (generalised anxiety disorder, obsessive compulsive disorder, panic disorder), bipolar disorder, addiction/impulse-control disorders and autism spectrum disorders. Neuropsychiatric symptoms (e.g. psychosis, anhedonia, agitation, etc) of neurodegenerative diseases (e.g. Alzheimer's disease, Parkinson's disease, Huntington's disease, etc) could also be treated by GPR52 agonists. GPR52 expression in the pituitary gland and hypothalamus suggests utility for GPR52 modulators in pituitary and hypothalamic disorders, and there is preclinical evidence (Xiong et al, 2016, WO2016/176571) to suggest that GPR52 agonists could be useful in the treatment of hyperprolactinemia. GPR52 modulators have been disclosed in WO2021/090030.

It is desirable to modify the physicochemical properties of GPR52 modulators.

## THE INVENTION

The present invention provides compounds that are prodrugs of G protein-coupled receptor 52 (GPR52) modulators.

Accordingly, the invention provides a compound of Formula (1a) or (1b):

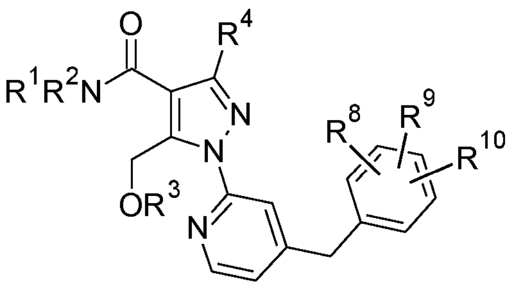

(1a);

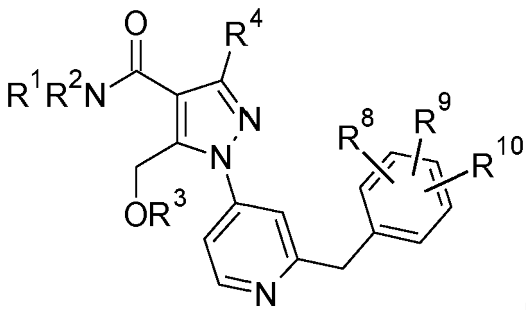

(1b);

or a salt thereof, wherein;

- $R^1$ and $R^2$ are independently H or $C_{1-3}$ alkyl optionally substituted with OH or 1 to 6 fluorine atoms; or $R^1$ and $R^2$ are joined to form a 4, 5, 6 or 7-membered ring which is optionally substituted with OH or 1 to 6 fluorine atoms;
- $R^3$ is a functional group which can be cleaved *in vivo* to afford a compound where $R^3$ is H;
- $R^4$ is H, CN, halo, or $C_{1-3}$ alkyl optionally substituted with OH or 1 to 6 fluorine atoms;
- $R^8$, $R^9$ and $R^{10}$ are independently H, CN, halo, or $C_{1-3}$ alkyl optionally substituted with 1 to 6 fluorine atoms.

Compounds of the present invention may be used as prodrugs of GPR52 modulators. Compounds of the present invention may be used as prodrugs of GPR52 agonists. Compounds of the present invention may be used in the manufacture of medicaments. The compounds or medicaments may be for use in treating, preventing, ameliorating,

controlling or reducing the risk of diseases or disorders in which GPR52 receptors are involved. The compounds or medicaments may be for use in treating, preventing, ameliorating, controlling or reducing the risk of diseases or disorders in which modulation of GPR52 receptors may be beneficial. Compounds of the present invention may be useful in the treatment of psychiatric disorders; neuropsychiatric disorders; neurodegenerative disorders; psychotic disorders; cognitive disorders; neurocognitive disorders; extrapyramidal disorders; movement disorders; motor disorders; hyperkinetic movement disorders; catatonia; mood disorders; depressive disorders; anxiety disorders; obsessive-compulsive disorder (OCD); autism spectrum disorders; depressive disorders; hypothalamic disorders; pituitary disorders; prolactin-related disorders; trauma- or stressor-related disorders; disruptive, impulse-control or conduct disorders; sleep-wake disorders; substance-related disorders; addictive disorders; behavioral disorders; hypofrontality; abnormalities in the tuberoinfundibular, mesolimbic, mesocortical, or nigrostriatal pathway; decreased activity in the striatum; cortical dysfunction; neurocognitive dysfunction or conditions or symptoms related thereto.

Compounds of the present invention may be useful in the treatment of schizophrenia, depression, attention-deficit hyperactivity disorder (ADHD), generalised anxiety disorder, obsessive-compulsive disorder (OCD), panic disorder, bipolar disorder, addiction/impulse-control disorders, autism spectrum disorders, psychosis, anhedonia, agitation, Alzheimer's disease, Parkinson's disease, Huntington's disease, vascular dementia, Lewy body disease, frontotemporal dementia, Tourette's syndrome, hyperprolactinemia, pituitary adenoma, prolactinoma, craniopharyngioma, Cushing's disease, diabetes insipidus, non-functioning tumours, obesity, posttraumatic stress disorder (PTSD), akathisia and associated movements, athetosis, ataxia, ballismus, hemiballismus, chorea, choreoathetosis, dyskinesia, tardive dyskinesia, neuroleptic-induced dyskinesia, myoclonus, mirror movement disorder, paroxysmal kinesigenic dyskinesia, restless legs syndrome, spasms, stereotypic movement disorder, sterotypy, Tic disorder, tremor, Wilson's disease, schizotypal personality disorder, delusional disorder, brief psychotic disorder, schizophreniform disorder, schizoaffective disorder, substance- or medication-induced psychotic disorder, delusions, hallucinations, disorganized thinking, grossly disorganized or abnormal motor behavior, catatonia, major depressive disorder, bipolar I disorder, bipolar II disorder, cyclothymic disorder, substance- or medication-induced bipolar and related disorders, bipolar and related disorders due to another medical condition, separation anxiety disorder, selective mutism, specific phobia, social anxiety disorder, panic disorder, agoraphobia, generalized anxiety disorder, substance- or medication-induced anxiety disorder, anxiety disorders due to another medical condition, delirium, major neurocognitive disorder, minor neurocognitive disorder, amnesia, dementia, developmental coordination disorder, stereotypic movement disorder, a post-stroke effect, dentatorubral-pallidoluysian atrophy, diminished emotional expression, avolition, alogia and asociality.

## DETAILED DESCRIPTION OF THE INVENTION

The invention relates to novel compounds. The novel compounds of the invention are prodrugs of GPR52 modulators. The invention also relates to the use of novel compounds as prodrugs of modulators of the GPR52 receptor. The invention also relates to the use of novel compounds as prodrugs of agonists of the GPR52 receptor. The invention further relates to the use of novel compounds in the manufacture of medicaments for use as GPR52 modulators. Compounds of the present invention are prodrugs of GPR52 agonists. The compounds or medicaments may be for use in treating, preventing, ameliorating, controlling or reducing the risk of diseases or disorders in which GPR52 receptors are involved. The compounds or medicaments may be for use in treating, preventing, ameliorating, controlling or reducing the risk of diseases or disorders in which modulation of GPR52 receptors may be beneficial.

The invention further relates to compounds, compositions and medicaments that may be useful in the treatment of psychiatric disorders; neuropsychiatric disorders; neurodegenerative disorders; psychotic disorders; cognitive disorders; neurocognitive disorders; extrapyramidal disorders; movement disorders; motor disorders; hyperkinetic movement disorders; catatonia; mood disorders; depressive disorders; anxiety disorders; obsessive-compulsive disorder (OCD); autism spectrum disorders; depressive disorders; prolactin-related disorders; trauma- or stressor-related disorders; disruptive, impulse-control or conduct disorders; sleep-wake disorders; substance-related disorders; addictive disorders; behavioral disorders; hypofrontality; abnormalities in the tuberoinfundibular, mesolimbic, mesocortical, or nigrostriatal pathway; decreased activity in the striatum; cortical dysfunction; neurocognitive dysfunction or conditions or symptoms related thereto.

The invention provides a compound of Formula (1a) or (1b):

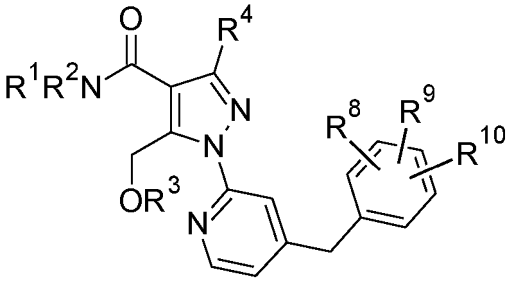 (1a); 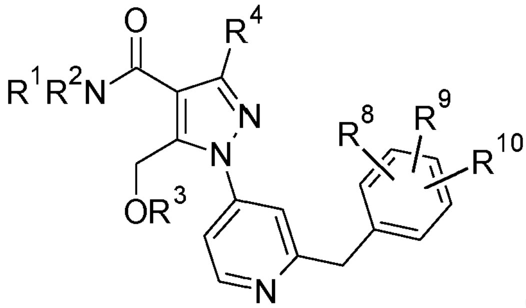 (1b);

or a salt thereof, wherein;

$R^1$ and $R^2$ are independently H or $C_{1-3}$ alkyl optionally substituted with OH or 1 to 6 fluorine atoms; or $R^1$ and $R^2$ are joined to form a 4, 5, 6 or 7-membered ring which is optionally substituted with OH or 1 to 6 fluorine atoms;
$R^3$ is a functional group which can be cleaved *in vivo* to afford a compound where $R^3$ is H; $R^4$ is H, CN, halo, or $C_{1-3}$ alkyl optionally substituted with OH or 1 to 6 fluorine atoms;
$R^8$, $R^9$ and $R^{10}$ are independently H, CN, halo, or $C_{1-3}$ alkyl optionally substituted with 1 to 6 fluorine atoms.

The invention provides a compound of Formula (1a) or (1b):

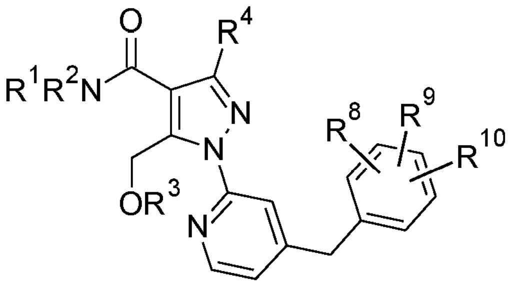 (1a); 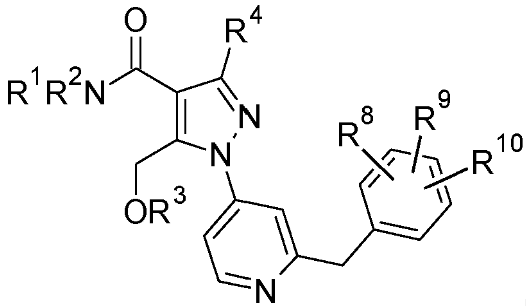 (1b);

or a salt thereof, wherein;

$R^1$ and $R^2$ are independently H or $C_{1-3}$ alkyl optionally substituted with OH or 1 to 6 fluorine atoms; or $R^1$ and $R^2$ are joined to form a 4, 5, 6 or 7-membered ring which is optionally substituted with OH or 1 to 6 fluorine atoms;
$R^3$ is $-P(O)OR^6OR^7$ or $-COR^5$;
$R^4$ is H, CN, halo, or $C_{1-3}$ alkyl optionally substituted with OH or 1 to 6 fluorine atoms;
$R^5$ is optionally substituted $C_{1-6}$ alkyl, optionally substituted 3- to 6-membered heterocyclyl or optionally substituted $C_{3-6}$ cycloalkyl;
$R^6$ and $R^7$ are independently H, optionally substituted $C_{1-6}$ alkyl, optionally substituted 3- to 6-membered heterocyclyl or optionally substituted $C_{3-6}$ cycloalkyl; or $R^6$ and $R^7$ are joined to form an optionally substituted 5- or 6-membered ring;
$R^8$, $R^9$ and $R^{10}$ are independently H, CN, halo, or $C_{1-3}$ alkyl optionally substituted with 1 to 6 fluorine atoms.

In the compounds herein, $R^1$ and $R^2$ can be independently H or $C_{1-3}$ alkyl optionally substituted with OH or 1 to 6 fluorine atoms. $R^1$ and $R^2$ can be joined to form a 4, 5, 6 or 7-membered ring which is optionally substituted with OH or 1 to 6 fluorine atoms. $R^1$ and $R^2$ can both be H. $R^1$ and $R^2$ can both be methyl. $R^1$ can be methyl and $R^2$ can be H.

$R^1$ can be H or $C_{1-3}$ alkyl optionally substituted with OH or 1 to 6 fluorine atoms. $R^1$ can be H. $R^1$ can be $C_{1-3}$ alkyl optionally substituted with OH or 1 to 6 fluorine atoms. $R^1$ can be $C_{1-3}$ alkyl. $R^1$ can be methyl. $R^1$ can be ethyl. $R^1$ can be n-propyl. $R^1$ can be isopropyl. $R^1$ can be $CH_2CH_2OH$. $R^1$ can be joined to $R^2$ to form a 4, 5, 6 or 7-membered ring which is optionally substituted with OH or 1 to 6 fluorine atoms. $R^1$ can be joined to $R^2$ to form a 4, 5, 6 or 7-membered carbocyclic ring which is optionally substituted with OH or 1 to 6 fluorine atoms. $R^1$ can be joined to $R^2$ to form a 4, 5, 6 or 7-membered carbocyclic ring. $R^1$ can be joined to $R^2$ to form an azetidine ring. $R^1$ can be joined to $R^2$ to form a pyrrolidine ring. $R^1$ can be joined to $R^2$ to form a piperidine ring. $R^1$ can be joined to $R^2$ to form an azepane ring.

$R^2$ can be H or $C_{1-3}$ alkyl optionally substituted with OH or 1 to 6 fluorine atoms. $R^2$ can be H. $R^2$ can be $C_{1-3}$ alkyl optionally substituted with OH or 1 to 6 fluorine atoms. $R^2$ can be $C_{1-3}$ alkyl. $R^2$ can be methyl. $R^2$ can be ethyl. $R^2$ can be n-

propyl. $R^2$ can be isopropyl. $R^2$ can be $CH_2CH_2OH$. $R^2$ can be joined to $R^1$ to form a 4, 5, 6 or 7-membered ring which is optionally substituted with OH or 1 to 6 fluorine atoms. $R^2$ can be joined to $R^1$ to form a 4, 5, 6 or 7-membered carbocyclic ring which is optionally substituted with OH or 1 to 6 fluorine atoms. $R^2$ can be joined to $R^1$ to form a 4, 5, 6 or 7-membered carbocyclic ring. $R^2$ can be joined to $R^1$ to form an azetidine ring. $R^2$ can be joined to $R^1$ to form a pyrrolidine ring. $R^2$ can be joined to $R^1$ to form a piperidine ring. $R^2$ can be joined to $R^1$ to form an azepane ring.

$R^3$ represents a prodrug moiety which can be cleaved *in vivo.* $R^3$ can be a functional group which can be cleaved *in vivo* to afford a compound where $R^3$ is H. Examples of such functional groups include groups that form carbonates, carbamates, esters, phosphates and phosphonooxymethylethers with the O atom to which they are attached. Examples of specific groups that may be suitable can be found in Rautio, J. et al. Nat. Rev. Drug Discov. 17, 559-587 (2018).

$R^3$ can be $-P(O)OR^6OR^7$ or $-COR^5$. $R^3$ can be $-PO_3H_2$ or $-COR^5$. $R^3$ can be $-PO_3H_2$, or a salt thereof. $R^3$ can be $-PO_3H_2$. $R^3$ can be $-COR^5$. $R^3$ can be -COMe. $R^3$ can be -COEt. $R^3$ can be a salt of $-PO_3H_2$. $R^3$ can be $-PO_3HX$, $-PO_3X$ or $-PO_3X_2$, where X is a suitable counter-ion. X can be any suitable counter-ion, including but not limited to those of Na, K, Li, Mg, Ca.

$R^4$ can be H, CN, halo, or $C_{1-3}$ alkyl optionally substituted with OH or 1 to 6 fluorine atoms. $R^4$ can be H. $R^4$ can be CN. $R^4$ can be halo. $R^4$ can be Cl. $R^4$ can be F. $R^4$ can be Br. $R^4$ can be $C_{1-3}$ alkyl optionally substituted with OH or 1 to 6 fluorine atoms. $R^4$ can be $C_{1-3}$ alkyl. $R^4$ can be methyl. $R^4$ can be ethyl. $R^4$ can be n-propyl. $R^4$ can be isopropyl. $R^4$ can be $CH_2OH$. $R^4$ can be $CF_3$. $R^4$ can be $CHF_2$.

$R^5$ can be optionally substituted $C_{1-6}$ alkyl, optionally substituted 3- to 6-membered heterocyclyl or optionally substituted $C_{3-6}$ cycloalkyl. $R^5$ can be $C_{1-6}$ alkyl optionally substituted with 1 to 6 fluorine atoms. $R^5$ can be methyl. $R^5$ can be ethyl.

$R^6$ and $R^7$ can independently be H, optionally substituted $C_{1-6}$ alkyl, optionally substituted 3-to 6-membered heterocyclyl or optionally substituted $C_{3-6}$ cycloalkyl; or $R^6$ and $R^7$ can be joined to form an optionally substituted 5- or 6-membered ring. $R^6$ and $R^7$ can together represent any such functional group that is suitable for use in phosphate prodrugs. Groups such as those described in Top. Curr. Chem. 2015; 360; 115-160, for example may be suitable. $R^6$ and $R^7$ can independently be H or $C_{1-6}$ alkyl optionally substituted with 1 to 6 fluorine atoms. $R^6$ and $R^7$ can both be H.

$R^6$ can be H, optionally substituted $C_{1-6}$ alkyl, optionally substituted 3- to 6-membered heterocyclyl or optionally substituted $C_{3-6}$ cycloalkyl; or $R^6$ can be joined to $R^7$ to form an optionally substituted 5- or 6-membered ring. $R^6$ can be $C_{1-6}$ alkyl optionally substituted with 1 to 6 fluorine atoms. $R^6$ can be methyl. $R^6$ can be ethyl. $R^6$ can be H.

$R^7$ can be H, optionally substituted $C_{1-6}$ alkyl, optionally substituted 3- to 6-membered heterocyclyl or optionally substituted $C_{3-6}$ cycloalkyl; or $R^7$ can be joined to $R^3$ to form an optionally substituted 5- or 6-membered ring. $R^7$ can be $C_{1-6}$ alkyl optionally substituted with 1 to 6 fluorine atoms. $R^7$ can be methyl. $R^7$ can be ethyl. $R^7$ can be H.

$R^8$, $R^9$ and $R^{10}$ can independently be H, CN, halo, or $C_{1-3}$ alkyl optionally substituted with 1 to 6 fluorine atoms. $R^8$, $R^9$ and $R^{10}$ can be independently selected from H, F, $CHF_2$ and $CF_3$.

$R^8$ can be H, CN, halo, or $C_{1-3}$ alkyl optionally substituted with 1 to 6 fluorine atoms. $R^8$ can be H, F, $CHF_2$ or $CF_3$. $R^8$ can be H. $R^8$ can be F. $R^8$ can be $CHF_2$. $R^8$ can be $CF_3$.

$R^9$ can be H, CN, halo, or $C_{1-3}$ alkyl optionally substituted with 1 to 6 fluorine atoms. $R^9$ can be H, F, $CHF_2$ or $CF_3$. $R^9$ can be H. $R^9$ can be F. $R^9$ can be $CHF_2$. $R^9$ can be $CF_3$.

$R^{10}$ can be H, CN, halo, or $C_{1-3}$ alkyl optionally substituted with 1 to 6 fluorine atoms. $R^{10}$ can be H, F, $CHF_2$ or $CF_3$. $R^{10}$ can be H. $R^{10}$ can be F. $R^{10}$ can be $CHF_2$. $R^{10}$ can be $CF_3$.

The group:

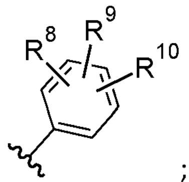

;

can be:

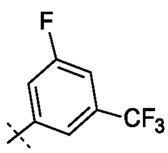

; or

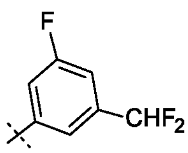

.

The compound can be a compound of formula (2a), (2b), (2c) or (2d):

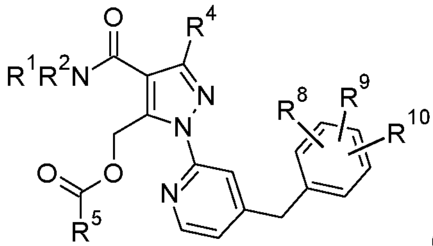 (2a); 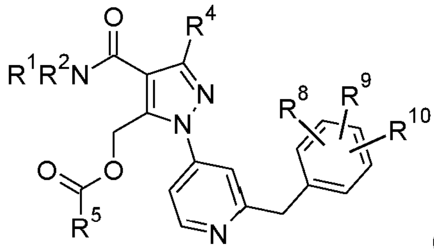 (2b);

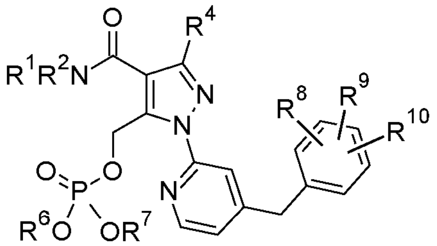 (2c); 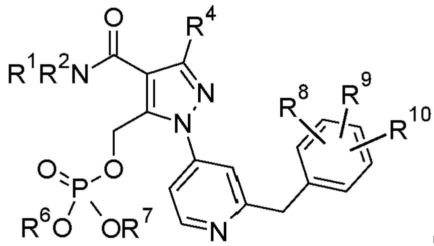 (2d);

or a salt thereof, wherein $R^1$, $R^2$, $R^4$, $R^5$, $R^6$, $R^7$, $R^8$, $R^9$ and $R^{10}$ are as defined above.

The compound can be a compound of formula (3):

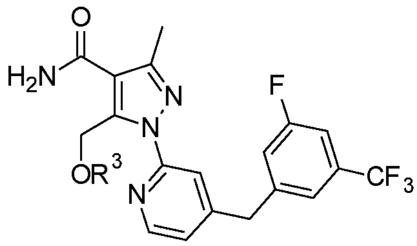 (3);

or a salt thereof, wherein $R^3$ is as defined above.

The compound can be a compound of formula (4a) or (4b):

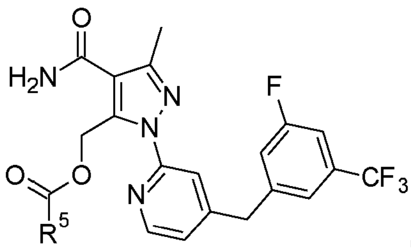 (4a); 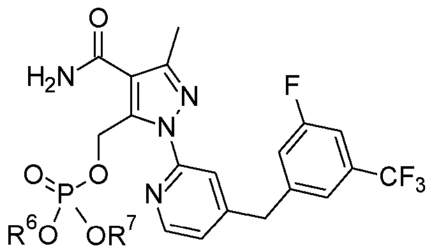 (4b);

or a salt thereof, wherein $R^5$, $R^6$ and $R^7$ are as defined above.

Upon administration, the prodrug compounds herein are metabolised into a compound which is particularly active as a GPR52 modulator. Metabolism may involve cleavage of the group comprising $R^3$. In particular the metabolised compound may be active as a GPR52 agonist.

Said metabolism of compounds of Formula (1a) and (1b) described above may comprise conversion to compounds of Formula (1ai) and (1bi) respectively.

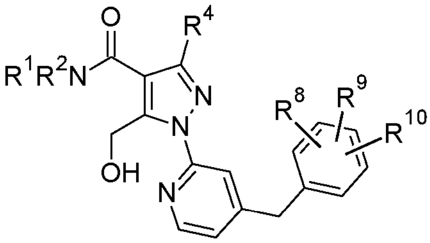

(1ai);

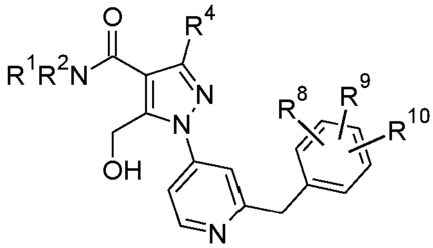

(1bi).

Compounds of Formula (1ai) and (1bi) are particularly active as GPR52 modulators, in particular as agonists of GPR52, as demonstrated in Applicant's earlier application PCT/GB2021/050638 (WO2021/181122A1).

The compounds can be prodrugs of 1-(4-(3-fluoro-5-(trifluoromethyl)benzyl)pyridin-2-yl)-5-(hydroxymethyl)-3-methyl-1*H*-pyrazole-4-carboxamide.

The compounds can be prodrugs of:

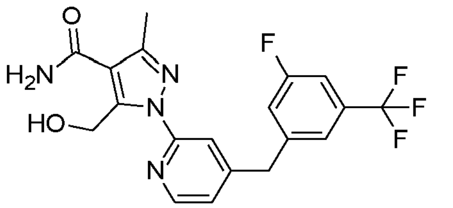

.

The compound can be selected from the group consisting of:

(4-carbomyl-1-(4-(3-fluoro-5-(trifluoromethyl)benzyl)pyridine-2-yl)-3-methyl-1*H*-pyrazol-5-yl) methyl acetate;
(4-carbamoyl-1-(4-(3-fluoro-5-(trifluoromethyl)benzyl)pyridine-2-yl)-3-methyl-1*H*-pyrazol-5-yl) dihydrogen phosphate;
or a salt thereof.

The compound can be selected from the group consisting of:

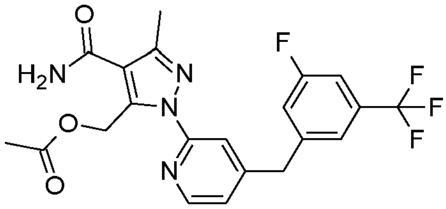

;

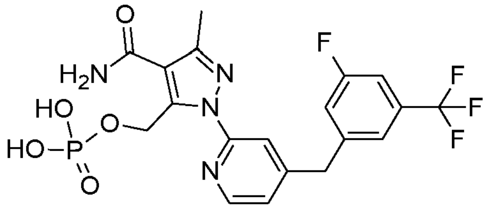

;

or a salt thereof.

The compounds can be prodrugs of:

1-(4-(3-fluoro-5-(trifluoromethyl)benzyl)pyridin-2-yl)-N-(2-hydroxyethyl)-5-(hydroxymethyl)-3-methyl-1H-pyrazole-4-carboxamide;
1-(2-(3-fluoro-5-(trifluoromethyl)benzyl)pyridin-4-yl)-5-(hydroxymethyl)-3-methyl-1H-pyrazole-4-carboxamide;
1-(2-(3-fluoro-5-(trifluoromethyl)benzyl)pyridin-4-yl)-N-(2-hydroxyethyl)-5-(hydroxymethyl)-3-methyl-1H-pyrazole-4-carboxamide;
1-(4-(3-fluoro-5-(trifluoromethyl)benzyl)pyridin-2-yl)-5-(hydroxymethyl)-N,3-dimethyl-1H-pyrazole-4-carboxamide; or
1-(4-(3-(difluoromethyl)-5-fluorobenzyl)pyridin-2-yl)-5-(hydroxymethyl)-3-methyl-1H-pyrazole-4-carboxamide.

The compounds can be prodrugs of:

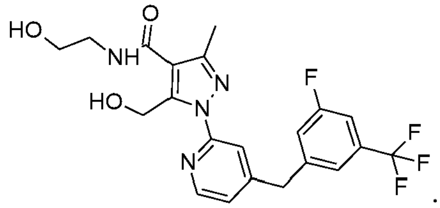
;

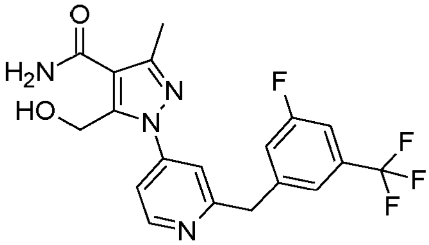
;
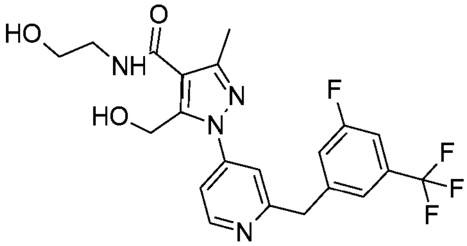
;

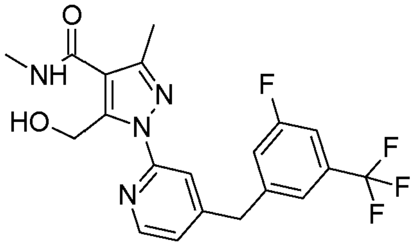
; or
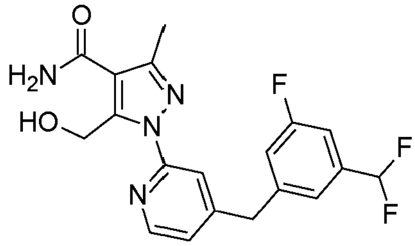
.

The compound can be selected from the group consisting of:

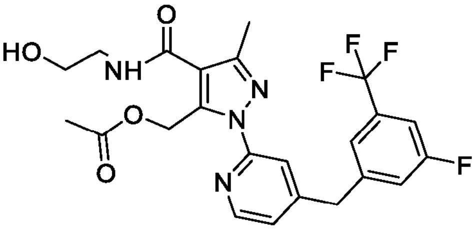
;
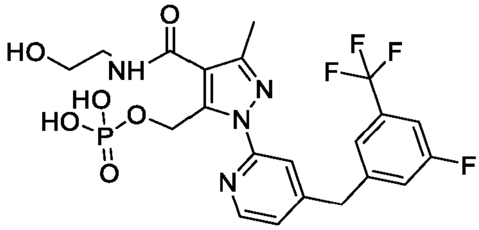
;

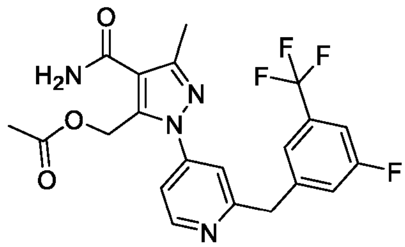
;
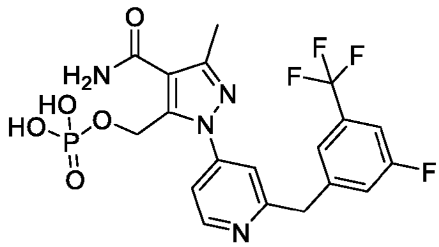
;

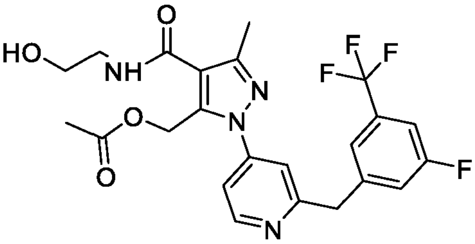
;
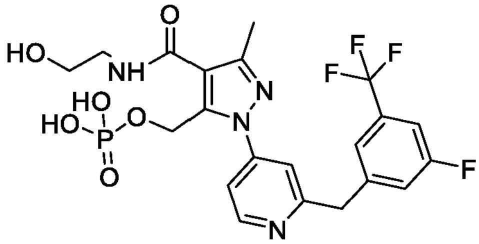
;

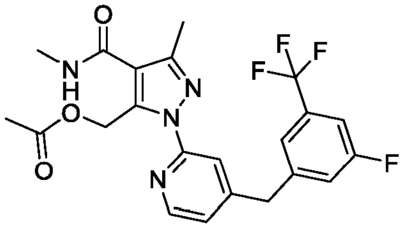

;

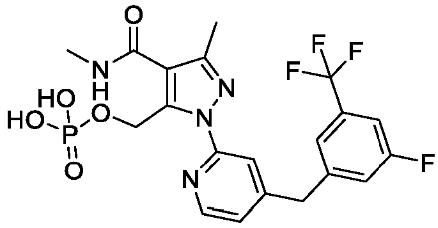

;

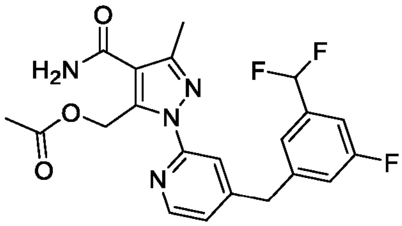

;

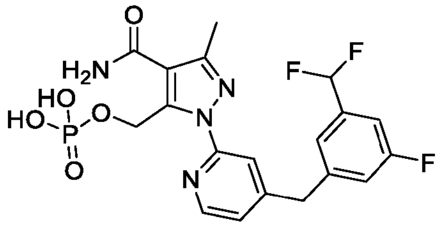

;

or a salt thereof.

The compound can be selected from the group consisting of:

(1-(4-(3-fluoro-5-(trifluoromethyl)benzyl)pyridin-2-yl)-4-((2-hydroxyethyl)carbamoyl)-3-methyl-1H-pyrazol-5-yl)methyl acetate;
(4-carbamoyl-1-(2-(3-fluoro-5-(trifluoromethyl)benzyl)pyridin-4-yl)-3-methyl-1H-pyrazol-5-yl)methyl acetate;
(1-(2-(3-fluoro-5-(trifluoromethyl)benzyl)pyridin-4-yl)-4-((2-hydroxyethyl)carbamoyl)-3-methyl-1H-pyrazol-5-yl)methyl acetate;
(1-(4-(3-fluoro-5-(trifluoromethyl)benzyl)pyridin-2-yl)-3-methyl-4-(methylcarbamoyl)-1H-pyrazol-5-yl)methyl acetate;
(4-carbamoyl-1-(4-(3-(difluoromethyl)-5-fluorobenzyl)pyridin-2-yl)-3-methyl-1H-pyrazol-5-yl)methyl acetate;
(1-(4-(3-fluoro-5-(trifluoromethyl)benzyl)pyridin-2-yl)-4-((2-hydroxyethyl)carbamoyl)-3-methyl-1H-pyrazol-5-yl)methyl dihydrogen phosphate;
(4-carbamoyl-1-(2-(3-fluoro-5-(trifluoromethyl)benzyl)pyridin-4-yl)-3-methyl-1H-pyrazol-5-yl)methyl dihydrogen phosphate;
(1-(2-(3-fluoro-5-(trifluoromethyl)benzyl)pyridin-4-yl)-4-((2-hydroxyethyl)carbamoyl)-3-methyl-1H-pyrazol-5-yl)methyl dihydrogen phosphate;
(1-(4-(3-fluoro-5-(trifluoromethyl)benzyl)pyridin-2-yl)-3-methyl-4-(methylcarbamoyl)-1H-pyrazol-5-yl)methyl dihydrogen phosphate;
(4-carbamoyl-1-(4-(3-(difluoromethyl)-5-fluorobenzyl)pyridin-2-yl)-3-methyl-1H-pyrazol-5-yl)methyl dihydrogen phosphate;
or a salt thereof.

Further embodiments of the invention include the use of a compound of Formula (1a), (1b), (2a), (2b), (2c), (2d), (3), (4a), (4b) or a salt thereof or a pharmaceutical composition comprising a compound of Formula (1a), (1b), (2a), (2b), (2c), (2d), (3), (4a), (4b) or a salt thereof in the treatment or prevention of diseases in which GPR52 receptors are involved or in which modulation of GPR52 receptors may be beneficial. Compounds of the present invention may be used as prodrugs of GPR52 modulators. Compounds of the present invention may be used as prodrugs of GPR52 agonists.

Compounds of the present invention may be used in the treatment of psychiatric disorders; neuropsychiatric disorders; neurodegenerative disorders; psychotic disorders; cognitive disorders; neurocognitive disorders; extrapyramidal disorders; movement disorders; motor disorders; hyperkinetic movement disorders; catatonia; mood disorders; depressive disorders; anxiety disorders; obsessive-compulsive disorder (OCD); autism spectrum disorders; depressive disorders; hypothalamic disorders; pituitary disorders; prolactin-related disorders; trauma- or stressor-related disorders; disruptive, impulse-control or conduct disorders; sleep-wake disorders; substance-related disorders; addictive disorders; behavioral disorders; hypofrontality; abnormalities in the tuberoinfundibular, mesolimbic, mesocortical, or nigrostriatal pathway; decreased activity in the striatum; cortical dysfunction; neurocognitive dysfunction or conditions or symptoms related thereto.

Compounds of the present invention may be used in the treatment of schizophrenia, depression, attention-deficit hyperactivity disorder (ADHD), generalised anxiety disorder, obsessive-compulsive disorder (OCD), panic disorder, bipolar disorder, addiction/impulse-control disorders, autism spectrum disorders, psychosis, anhedonia, agitation,

Alzheimer's disease, Parkinson's disease, Huntington's disease, vascular dementia, Lewy body disease, frontotemporal dementia, Tourette's syndrome, hyperprolactinemia, pituitary adenoma, prolactinoma, craniopharyngioma, Cushing's disease, diabetes insipidus, non-functioning tumours, obesity, posttraumatic stress disorder (PTSD), akathisia and associated movements, athetosis, ataxia, ballismus, hemiballismus, chorea, choreoathetosis, dyskinesia, tardive dyskinesia, neuroleptic-induced dyskinesia, myoclonus, mirror movement disorder, paroxysmal kinesigenic dyskinesia, restless legs syndrome, spasms, stereotypic movement disorder, sterotypy, Tic disorder, tremor, Wilson's disease, schizotypal personality disorder, delusional disorder, brief psychotic disorder, schizophreniform disorder, schizoaffective disorder, substance- or medication-induced psychotic disorder, delusions, hallucinations, disorganized thinking, grossly disorganized or abnormal motor behavior, catatonia, major depressive disorder, bipolar I disorder, bipolar II disorder, cyclothymic disorder, substance- or medication-induced bipolar and related disorders, bipolar and related disorders due to another medical condition, separation anxiety disorder, selective mutism, specific phobia, social anxiety disorder, panic disorder, agoraphobia, generalized anxiety disorder, substance- or medication-induced anxiety disorder, anxiety disorders due to another medical condition, delirium, major neurocognitive disorder, minor neurocognitive disorder, amnesia, dementia, developmental coordination disorder, stereotypic movement disorder, a post-stroke effect, dentatorubral-pallidoluysian atrophy, diminished emotional expression, avolition, alogia and asociality.

Compounds of the present invention may be used in the treatment of schizophrenia, depression, attention-deficit hyperactivity disorder (ADHD), generalised anxiety disorder, obsessive-compulsive disorder (OCD), panic disorder, bipolar disorder, addiction/impulse-control disorders, autism spectrum disorders, psychosis, neurocognitive disorder, delirium, anhedonia, agitation, Alzheimer's disease, Parkinson's disease, Huntington's disease, vascular dementia, Lewy body disease, frontotemporal dementia, Tourette's syndrome, hyperprolactinemia, obesity, and posttraumatic stress disorder (PTSD). Compounds of the present invention may be used in the treatment of schizophrenia.

## DEFINITIONS

In this application, the following definitions apply, unless indicated otherwise.

The term "GPR52 modulator" as used herein refers to any compound which binds to and modulates the function of the GPR52 receptor. The term "modulator" should be interpreted to include modulation by modalities including, but not limited to, agonists, partial agonists and inverse agonists.

The term "prodrug" as used herein refers to a compound which is metabolically or chemically activated *in vivo* to afford an active parent drug molecule which itself affords the therapeutic pharmacological effect. Prodrugs as described herein may be converted by one or more metabolic process *in vivo* to afford a compound which is active as a GPR52 modulator. Prodrugs as described herein may be converted by one or more metabolic process *in vivo* to afford a compound which is active as a GPR52 agonist. The unmetabolised prodrug compounds themselves may or may not have GPR52 activity. The use prodrugs is well precedented, and indeed many marketed drugs are prodrugs (Rautio, J. et al. Nat. Rev. Drug Discov. 17, 559-587 (2018)).

The term "treatment", in relation to the uses of any of the compounds described herein, including those of Formula (1a), (1b), (2a), (2b), (2c), (2d), (3), (4a), (4b) is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" (for example in relation to methods of treatment of a disease or condition) refers to an amount of the compound which is effective to produce a desired therapeutic effect. For example, if the condition is pain, then the effective therapeutic amount is an amount sufficient to provide a desired level of pain relief. The desired level of pain relief may be, for example, complete removal of the pain or a reduction in the severity of the pain.

Terms such as "alkyl" and "halo" are all used in their conventional sense (e.g. as defined in the IUPAC Gold Book), unless indicated otherwise. "optionally substituted" as applied to any group means that the said group may if desired be substituted with one or more substituents, which may be the same or different.

To the extent that any of the compounds described have chiral centres, the present invention extends to all optical isomers of such compounds, whether in the form of racemates or resolved enantiomers. The invention described herein relates to all crystal forms, solvates and hydrates of any of the disclosed compounds however so prepared. To the extent that any of the compounds disclosed herein have acid or basic centres such as carboxylates or amino groups, then all salt forms of said compounds are included herein. In the case of pharmaceutical uses, the salt should be seen as being a pharmaceutically acceptable salt.

Salts or pharmaceutically acceptable salts that may be mentioned include acid addition salts and base addition salts. Such salts may be formed by conventional means, for example by reaction of a free acid or a free base form of a compound with one or more equivalents of an appropriate acid or base, optionally in a solvent, or in a medium in which the salt is insoluble, followed by removal of said solvent, or said medium, using standard techniques (e.g. in vacuo, by freeze-drying or by filtration). Salts may also be prepared by exchanging a counter-ion of a compound in the form of a salt with

another counter-ion, for example using a suitable ion exchange resin.

Examples of pharmaceutically acceptable salts include acid addition salts derived from mineral acids and organic acids, and salts derived from metals such as sodium, magnesium, potassium and calcium.

Examples of acid addition salts include acid addition salts formed with acetic, 2,2-dichloroacetic, adipic, alginic, aryl sulfonic acids (e.g. benzenesulfonic, naphthalene-2-sulfonic, naphthalene-1,5-disulfonic and p-toluenesulfonic), ascorbic (e.g. L-ascorbic), L-aspartic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic), α-oxoglutaric, glycolic, hippuric, hydrobromic, hydrochloric, hydriodic, isethionic, lactic (e.g. (+)-L-lactic and (±)-DL-lactic), lactobionic, maleic, malic (e.g. (-)-L-malic), malonic, (±)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids.

Also encompassed are any solvates of the compounds and their salts. Preferred solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulfoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGA), differential scanning calorimetry (DSC) and X-ray crystallography.

The solvates can be stoichiometric or non-stoichiometric solvates. Particular solvates may be hydrates, and examples of hydrates include hemihydrates, monohydrates and dihydrates. For a more detailed discussion of solvates and the methods used to make and characterise them, see Bryn et al, Solid-State Chemistry of Drugs, Second Edition, published by SSCI, Inc of West Lafayette, IN, USA, 1999, ISBN 0-967-06710-3.

The term "pharmaceutical composition" in the context of this invention means a composition comprising an active agent and comprising additionally one or more pharmaceutically acceptable carriers. The composition may further contain ingredients selected from, for example, diluents, adjuvants, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The compositions may take the form, for example, of tablets, dragees, powders, elixirs, syrups, liquid preparations including suspensions, sprays, inhalants, tablets, lozenges, emulsions, solutions, cachets, granules, capsules and suppositories, as well as liquid preparations for injections, including liposome preparations.

The compounds of the invention may contain one or more isotopic substitutions, and a reference to a particular element includes within its scope all isotopes of the element. For example, a reference to hydrogen includes within its scope $^{1}H$, $^{2}H$ (D), and $^{3}H$ (T). Similarly, references to carbon and oxygen include within their scope respectively $^{12}C$, $^{13}C$ and $^{14}C$ and $^{16}O$ and $^{13}O$. In an analogous manner, a reference to a particular functional group also includes within its scope isotopic variations, unless the context indicates otherwise. For example, a reference to an alkyl group such as an ethyl group or an alkoxy group such as a methoxy group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group) or a methoxy group in which all three hydrogen atoms are in the deuterium isotopic form (a trideuteromethoxy group). The isotopes may be radioactive or non-radioactive.

Therapeutic dosages may be varied depending upon the requirements of the patient, the severity of the condition being treated, and the compound being employed. Determination of the proper dosage for a particular situation is within the skill of the art. Generally, treatment is initiated with the smaller dosages which are less than the optimum dose of the compound. Thereafter the dosage is increased by small increments until the optimum effect under the circumstances is reached. For convenience, the total daily dosage may be divided and administered in portions during the day if desired.

The magnitude of an effective dose of a compound will, of course, vary with the nature of the severity of the condition to be treated and with the particular compound and its route of administration. The selection of appropriate dosages is within the ability of one of ordinary skill in this art, without undue burden. In general, the daily dose range may be from about 10 μg to about 30 mg per kg body weight of a human and non-human animal, preferably from about 50 μg to about 30 mg per kg of body weight of a human and non-human animal, for example from about 50 μg to about 10 mg per kg of body weight of a human and non-human animal, for example from about 100 μg to about 30 mg per kg of body weight of a human and non-human animal, for example from about 100 μg to about 10 mg per kg of body weight of a human and non-human animal and most preferably from about 100 μg to about 1 mg per kg of body weight of a human and non-human animal.

## PHARMACEUTICAL FORMULATIONS

While it is possible for the active compound to be administered alone, it is preferable to present it as a pharmaceutical composition (e.g. formulation).

Accordingly, in some embodiments of the invention, there is provided a pharmaceutical composition comprising at least one compound of Formula (1a), (1b), (2a), (2b), (2c), (2d), (3), (4a), (4b) as defined above together with at least one pharmaceutically acceptable excipient.

The composition may be a tablet composition. The composition may be a capsule composition.

The pharmaceutically acceptable excipient(s) can be selected from, for example, carriers (e.g. a solid, liquid or semi-solid carrier), adjuvants, diluents (e.g solid diluents such as fillers or bulking agents; and liquid diluents such as solvents and co-solvents), granulating agents, binders, flow aids, coating agents, release-controlling agents (e.g. release retarding or delaying polymers or waxes), binding agents, disintegrants, buffering agents, lubricants, preservatives, anti-fungal and antibacterial agents, antioxidants, buffering agents, tonicity-adjusting agents, thickening agents, flavouring agents, sweeteners, pigments, plasticizers, taste masking agents, stabilisers or any other excipients conventionally used in pharmaceutical compositions.

The term "pharmaceutically acceptable" as used herein means compounds, materials, compositions, and/or dosage forms which are, within the scope of sound medical judgment, suitable for use in contact with the tissues of a subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation.

Pharmaceutical compositions containing compounds of the Formula (1a), (1b), (2a), (2b), (2c), (2d), (3), (4a), (4b) can be formulated in accordance with known techniques, see for example, Remington's Pharmaceutical Sciences, Mack Publishing Company, Easton, PA, USA. The pharmaceutical compositions can be in any form suitable for oral, parenteral, topical, intranasal, intrabronchial, sublingual, ophthalmic, otic, rectal, intra-vaginal, or transdermal administration.

Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches.

Tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Tablets may be designed to release the drug either upon contact with stomach fluids (immediate release tablets) or to release in a controlled manner (controlled release tablets) over a prolonged period of time or with a specific region of the GI tract.

The pharmaceutical compositions typically comprise from approximately 1% (w/w) to approximately 95% (w/w) active ingredient and from 99% (w/w) to 5% (w/w) of a pharmaceutically acceptable excipient (for example as defined above) or combination of such excipients. Preferably, the compositions comprise from approximately 20% (w/w) to approximately 90% (w/w) active ingredient and from 80% (w/w) to 10% of a pharmaceutically excipient or combination of excipients. The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, pre-filled syringes, dragées, powders, tablets or capsules.

Tablets and capsules may contain, for example, 0-20% disintegrants, 0-5% lubricants, 0-5% flow aids and/or 0-99% (w/w) fillers/ or bulking agents (depending on drug dose). They may also contain 0-10% (w/w) polymer binders, 0-5% (w/w) antioxidants, 0-5% (w/w) pigments. Slow release tablets would in addition typically contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers.

Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

The pharmaceutical formulations may be presented to a patient in "patient packs" containing an entire course of treatment in a single package, usually a blister pack.

The compounds of the Formula (1a), (1b), (2a), (2b), (2c), (2d), (3), (4a), (4b) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 milligrams to 1 gram, of active compound.

The active compound will be administered to a patient in need thereof (for example a human or animal patient) in an amount sufficient to achieve the desired therapeutic effect (effective amount). The precise amounts of compound administered may be determined by a supervising physician in accordance with standard procedures.

## EXAMPLES

The invention will now be illustrated, but not limited, by reference to the following examples shown in **Table 1.**

**Table 1 - Examples**

| | |
|---|---|
| 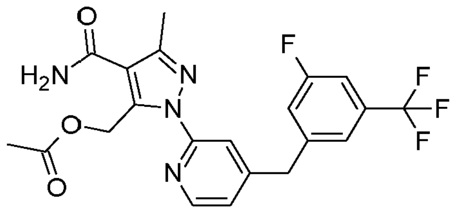<br>Example 1 | 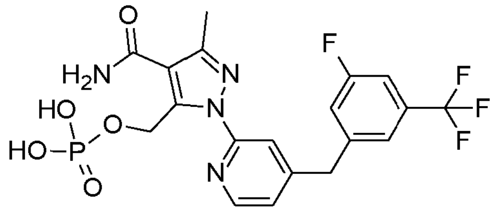<br>Example 2 |
| 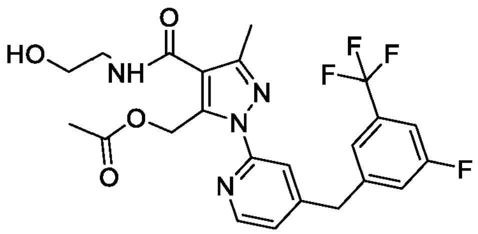<br>Example 3 | 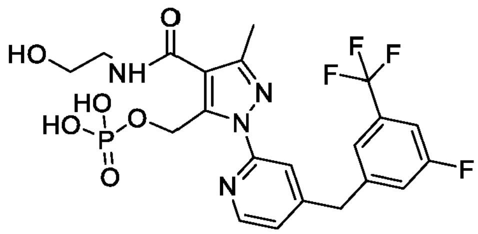<br>Example 4 |
| 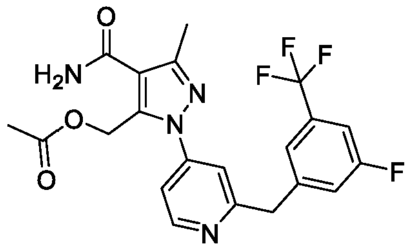<br>Example 5 | 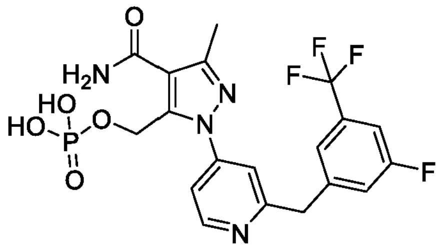<br>Example 6 |
| 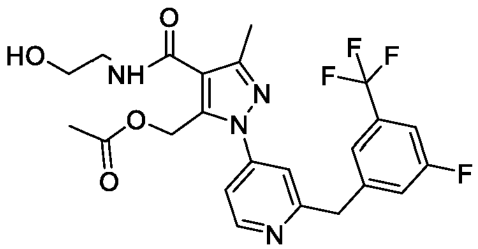<br>Example 7 | 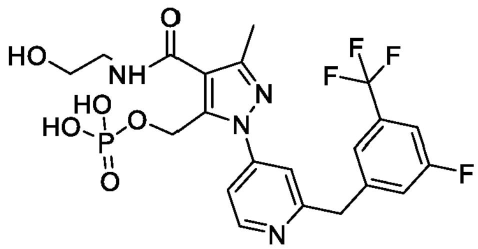<br>Example 8 |

(continued)

| | |
|---|---|
| 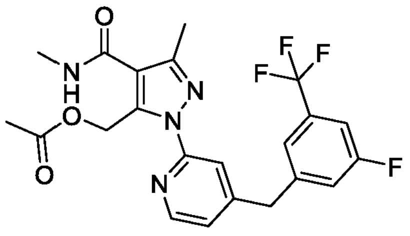 Example 9 | 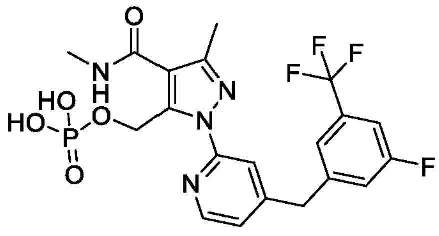 Example 10 |
| 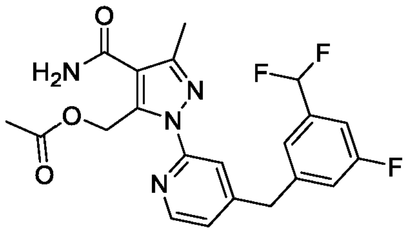 Example 11 | 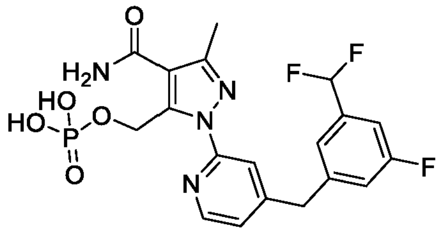 Example 12 |

## PREPARATION OF THE COMPOUNDS OF THE INVENTION

Compounds of Formula (1a), (1b), (2a), (2b), (2c), (2d), (3), (4a), (4b) can be prepared in accordance with synthetic methods known to the skilled person. The invention also provides a process for the preparation of a compound as defined in Formula (1a), (1b) and (2) above. Commercial reagents were utilized without further purification.

Abbreviations

| | |
|---|---|
| AcOH = | acetic acid |
| aq = | aqueous |
| DCM = | dichloromethane |
| DIPEA = | *N,N*-diisopropylethylamine |
| DMAP = | 4-dimethylaminopyridine |
| DMF = | *N,N*-dimethylformamide |
| DMSO = | dimethylsulfoxide |
| dppf = | 1,1'-ferrocenediyl-bis(diphenylphosphine) |
| EDCI = | 1-(3-dimethylaminopropyl)-3-ethylcarbodiimide hydrochloride |
| ES = | electrospray |
| EtOAc = | ethyl acetate |
| EtOH = | ethanol |
| h = | hour(s) |
| HATU = | *N*-[(Dimethylamino)-1*H*-1,2,3-triazolo-[4,5-*b*]pyridin-1-ylmethylene]-*N*-methylmethanaminium hexa- |

fluorophosphate *N*-oxide

L = litre

LC = liquid chromatography

LCMS = liquid chromatography mass spectrometry

MeCN = acetonitrile

MeOH = methanol

min = minute(s)

MS = mass spectrometry

NMR = nuclear magnetic resonance

Pet-ether = petroleum ether

RT = room temperature

TFA = trifluoroacetic acid

THF = tetrahydrofuran

UV = ultraviolet

LCMS methods

LCMS experiments were carried out using electrospray conditions under the conditions below (Solvents: A1 = 0.1% TFA in $H_2O$:MeCN (95:5); A2 = 5 mM ammonium acetate in $H_2O$; A3 = 2.5 L $H_2O$ + 2.5 mL 28% ammonia in $H_2O$ solution; A4 = 0.1% $HCO_2H$ in $H_2O$:MeCN (95:5); A5 = 10 mM $NH_4HCO_3$ in $H_2O$; A6 = 0.2% of 28% ammonia solution in $H_2O$; A7 = 0.1% TFA in $H_2O$; A8 = 50 mM ammonium acetate pH 7.4; A9 = 10mM ammonium acetate in $H_2O$; A10 = 0.1% formic acid in $H_2O$; B1 = 0.1% TFA in MeCN; B2 = MeCN; B3 = 2.5 L MeCN + 135 mL $H_2O$ + 2.5 mL 28% ammonia in $H_2O$ solution; B4 = 0.1% formic acid in MeCN. LCMS data are given in the format: Mass ion, electrospray mode (positive or negative), retention time; Mass ion, electrospray mode (positive or negative), retention time, approximate purity.

**Method 1.** Instruments: Agilent Technologies 1290 Infinity II Series LC, 6125 Quadrupole MSD SL; Column: Atlantis dC18 5 micron, 4.6 x 50mm; Gradient [time (min)/solvent B2 in A1 (%)]:0.0/5, 2.5/95, 4.0/95, 4.5/5, 6.0/5; Injection volume 1 μL; UV detection 210 to 400 nM; Column temperature 25 °C; 1.5 mL/min.

**Method 2.** Instruments: Agilent Technologies 1290 Infinity II Series LC, 6125 Quadrupole MSD SL; Column: Zorbax XDB C18, 5 micron; Gradient [time (min)/solvent B2 in A4 (%)]:0.00/5, 2.50/95, 4.00/95, 4.50/5, 6.00/5; Injection volume 1 μL; UV detection 210-400 nm; Column temperature 25 °C; Flow rate 1.5 mL/min.

**Method 3.** Instruments: Agilent Technologies 1290 Infinity II Series LC, 6125 Quadrupole MSD SL; Column: Waters XBridgeC8 3.5 micron, 4.6 x 50 mm; Gradient [time (min)/solvent B1 in A1 (%)]:0.0/5, 2.5/95, 4.0/95, 4.5/5, 6.0/5; Injection volume 1 μL; UV detection 210 to 400 nM; Column temperature 25 °C; 1.5 mL/min.

**Method 4.** Instruments: Agilent Technologies 1260 LC with Chemstation software, Diode Array Detector, Agilent 6120 Quadrupole MS with APCI and ES Source; Column: Phenomenex Gemini-NX C18, 3 micron, 2 x 30 mm; Gradient [time (min)/solvent B3 in A3 (%)]:0.00/5, 2.00/95, 2.50/95, 2.60/5, 3.00/5; Injection volume 0.5 μL; UV detection 190-400 nm; column temperature 40 °C; Flow rate 1.5 mL/min.

**Method 5.** Instruments: Agilent Technologies 1260 LC with Chemstation software, Diode Array Detector, Agilent 6120 Quadrupole MS with APCI and ES Source; Column: Phenomenex Gemini-NX C18, 3 micron, 2 x 30 mm; Gradient [time (min)/solvent B3 in A3 (%)]:0.00/2, 0.10/2, 8.40/95, 10.0/95, 10.1/2, 12.0/2; Injection volume 0.5 μL; UV detection 190-400 nm; column temperature 40 °C; Flow rate 1.5 mL/min.

**Method 6.** Instruments: Waters Acquity H-Class UPLC MS system with MassLynx software, Photo Diode Array Detector (PDA), QDa Mass detector with Electrospray source; Column: Phenomenex Luna PFP, 5 micron, 2.1 x 50 mm; Gradient [time (min)/solvent B4 in A10 (%)]: 0.0/ 5, 0.1/ 5, 8.5/ 95, 8.8/ 100, 9.6/ 100, 9.8/ 5, 10.0/ 5; Injection volume 1 μL; UV detection 200-400 nm; column temperature 40 °C; Flow rate 0.5 mL/min.

**Method 7.** Instruments: Agilent Technologies 1290 Infinity II Series LC, 6125 Quadrupole MSD SL; Column: Waters XBridgeC8 3.5 micron, 4.6 x 50 mm; Gradient [time (min)/solvent B2 in A5 (%)]:0.0/10, 4.0/95, 5.0/95, 5.5/10, 7.0/10.; Injection volume 1 μL; UV detection 210 to 400 nM; Column temperature 25 °C; 1.2 mL/min.

GCMS methods

GCMS data are given in the format: Mass ion, electrospray mode (positive or negative), retention time.

**Method 1.** Instrument: Agilent GCMS 7890B; Column: HP-5ms UI (30m x 250μm x 0.25μm); Inlet temp: 250 °C; Split ratio: 75:1; Oven temp: 50 °C, hold time 3 min; Ramp 1: 40 °C/min to 300 °C, hold time 2 min; Detector temperature: 310 °C; Column flow: 2 mL/min; Air flow: 300 mL/min; $H_2$ flow: 40 mL/min; Make up flow (He): 25 mL/min; Source temp: 230 °C.

**Method 2.** Instrument: Agilent GCMS 7890B; Column: HP-5ms UI (30m x 250μm x 0.25μm); Inlet temp: 250 °C; Split ratio: 75:1; Oven temp: 120 °C, hold time 1 min; Ramp 1: 40 °C/min to 300 °C, hold time 4 min; Detector temperature: 310 °C; Column flow: 2 mL/min; Air flow: 300 mL/min; $H_2$ flow: 40 mL/min; Make up flow (He): 25 mL/min; Source temp: 230 °C.

**PREPARATION OF INTERMEDIATES**

**Scheme 1**

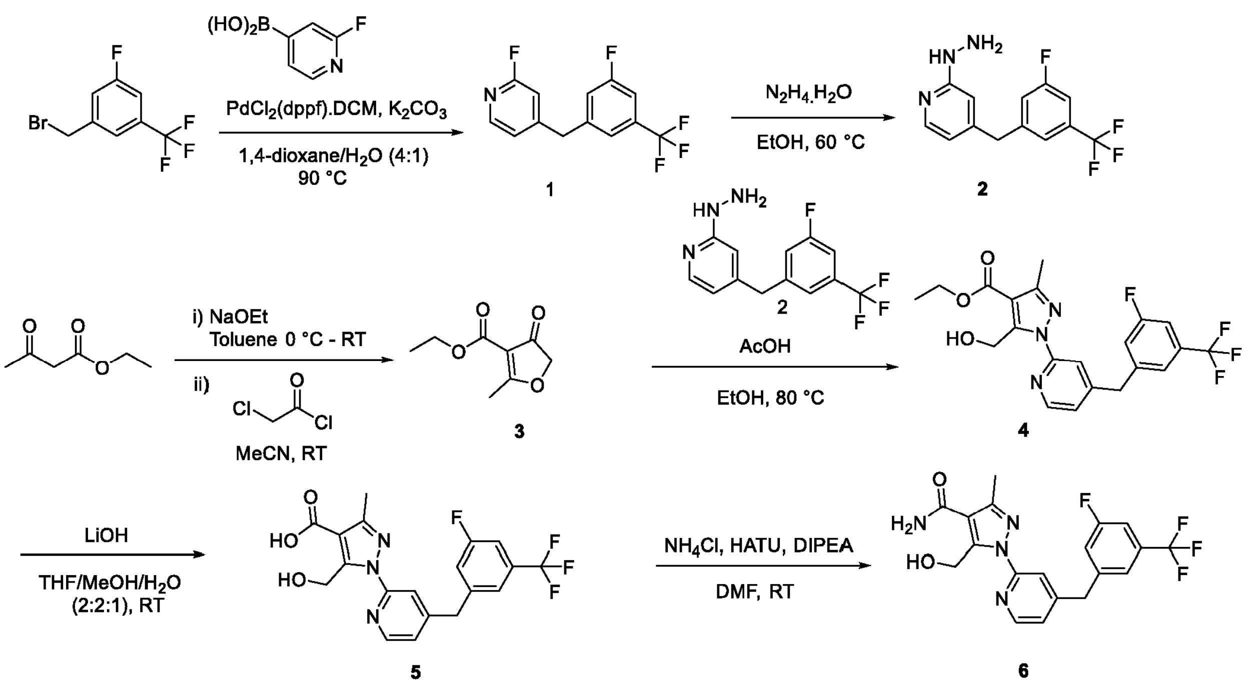


**Preparation of Intermediate 1 - 2-fluoro-4-(3-fluoro-5-(trifluoromethyl)benzyl)pyridine**

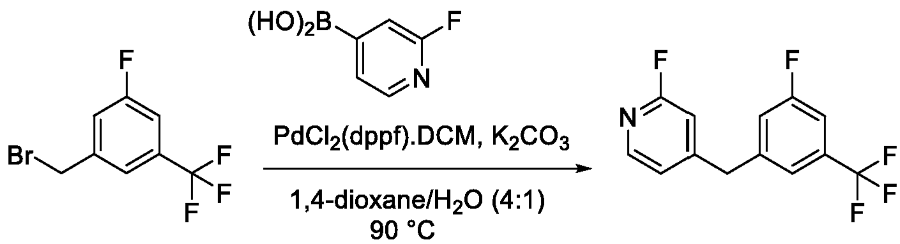

$PdCl_2$(dppf).DCM (284 mg, 0.38 mmol) was added to a degassed solution of (2-fluoropyridin-4-yl)boronic acid (1.3 g, 7.77 mmol), potassium carbonate (3.2 g, 22.3 mmol) and 1-(bromomethyl)-3-fluoro-5-(trifluoromethyl)benzene (1.3 g, 9.30 mmol) in 1,4-dioxane (20 mL)/water (5 mL) and the resultant reaction mixture heated at 90 °C for 1 h. The reaction mixture was partitioned between water (70 mL) and EtOAc (100 mL). The organic layer was separated, washed with brine (50 mL), dried ($Na_2SO_4$) and the solvent removed *in vacuo.* The residue was purified by gradient flash column chromatography eluting with 0-30% EtOAc in pet-ether to afford 2-fluoro-4-(3-fluoro-5-(trifluoromethyl)benzyl)pyridine as a brown oil (1.8 g, 86%).

**LCMS (Method 2):** m/z 274.0 $(M+H)^+$ (ES+), at 2.54 min.

$^1$**H NMR:** (400 MHz, DMSO-$d_6$) δ: 8.17 (d, *J* = 4.4 Hz, 1H), 7.61 (s, 3H), 7.29 (d, *J* = 1.2 Hz, 1H), 7.16 (s, 1H), 4.17 (d, *J* = 3.6 Hz, 2H).

**Preparation of Intermediate 2 - 4-(3-fluoro-5-(trifluoromethyl)benzyl)-2-hydrazineylpyridine**

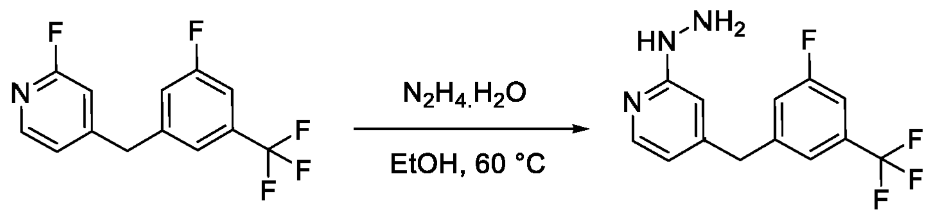


Hydrazine hydrate (0.17 mL, 3.52 mmol) was added to a stirred solution of 2-fluoro-4-(3-fluoro-5-(trifluoromethyl) benzyl)pyridine (Intermediate 1, 300 mg, 1.17 mmol) in EtOH (10 mL) and the resultant reaction mixture was heated at 60 °C for 16 h. The solvent was removed *in vacuo* and the residue partitioned between water (50 mL) and EtOAc (50 mL). The organic layer was separated, washed with brine (50 mL), dried ($Na_2SO_4$) and the solvent removed *in vacuo* to afford 4-(3-fluoro-5-(trifluoromethyl)benzyl)-2-hydrazineylpyridine as a brown oil (350 mg, 100%).

**LCMS (Method 2):** m/z 286.2 $(M+H)^+$ (ES+), at 1.37 min.

$^1$**H NMR:** (400 MHz, DMSO-$d_6$) δ: 7.88 (d, *J* = 5.2 Hz, 1H), 7.53 - 7.44 (m, 3H), 7.36 (s, 1H), 6.57 (s, 1H), 6.45 - 6.44 (m, 1H), 4.07 (s, 2H), 3.95 (s, 2H).

**Preparation of Intermediate 3 - ethyl 2-methyl-4-oxo-4,5-dihydrofuran-3-carboxylate**

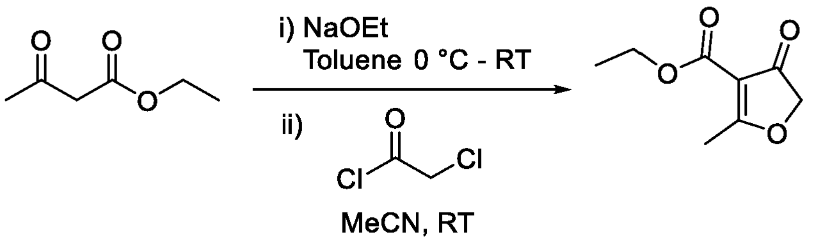


Sodium ethoxide (7.84 g, 115 mmol) was added and to a suspension of ethyl 3-oxobutanoate (10 g, 76.8 mmol) in toluene (50 mL) at 0 °C and the reaction mixture stirred at RT for 1 h. MeCN (20 mL) and 2-chloroacetyl chloride (6.15 mL, 38.4 mmol) were added and the resultant reaction mixture was stirred at RT for 2 h. The reaction mixture was acidified with 6 N aq $H_2SO_4$ (60 mL), the organic layer removed and the aqueous layer extracted with EtOAc (2x100 mL). The combined organic layers were washed with brine (100 mL), dried ($Na_2SO_4$) and the solvent removed *in vacuo.* The residue was purified by gradient flash column chromatography eluting with 0-10% EtOAc in pet-ether to afford ethyl 2-methyl-4-oxo-4,5-dihydrofuran-3-carboxylate as a yellow liquid (2.8 g, 21%).

**LCMS (Method 3):** m/z 171.0 $(M+H)^+$ (ES+), at 1.94 min.

$^1$**H NMR:** (400 MHz, DMSO-$d_6$) δ: 4.78 (s, 2H), 4.17 (q, *J* = 3.6 Hz, 2H), 2.50 (s, 3H), 1.22 (t, *J* = 9.6 Hz, 3H).

**Preparation of Intermediate 4 - ethyl 1-(4-(3-fluoro-5-(trifluoromethyl)benzyl)pyridin-2-yl)-5-(hydroxymethyl)-3-methyl-1*H*-pyrazole-4-carboxylate**

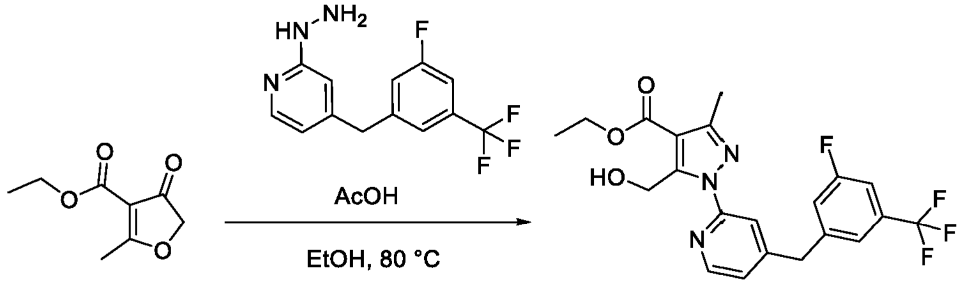


4-(3-Fluoro-5-(trifluoromethyl)benzyl)-2-hydrazineylpyridine (Intermediate 2, 4.69 g, 16.5 mmol) was added to a stirred solution of ethyl 2-methyl-4-oxo-4,5-dihydrofuran-3-carboxylate (Intermediate 3, 2.8 g, 16.5 mmol) in ethanol (50 mL) at RT followed by the addition of catalytic amount of acetic acid (0.094 mL, 1.65 mmol) and the resultant reaction mixture was heated at 80 °C for 16 h. On cooling, the solid which had come out of solution was filtered, rinsed with EtOH (2x10 mL) and dried *in vacuo* to afford ethyl 1-(4-(3-fluoro-5-(trifluoromethyl)benzyl)pyridin-2-yl)-5-(hydroxymethyl)-3-methyl-1*H*-pyrazole-4-carboxylate as a white solid (1.95 g, 27%).

**LCMS (Method 2):** m/z 438.0 $(M+H)^+$ (ES+), at 2.80 min.

**$^1$H NMR:** (400 MHz, DMSO-$d_6$) δ: 8.48 (d, *J* = 7.2 Hz, 1H), 7.84 (s, 1H), 7.67 (s, 1H), 7.61-7.59 (m, 2H), 7.43 (d, *J* = 6.4 Hz, 1H), 5.27 (t, *J* = 9.2 Hz, 1H), 5.01 (d, *J* = 8.8 Hz, 2H), 4.29-4.27 (m, 4H), 2.42 (s, 3H), 1.32 (t, *J* = 9.6 Hz, 3H).

**Preparation of Intermediate 5 - 1-(4-(3-fluoro-5-(trifluoromethyl)benzyl)pyridin-2-yl)-5-(hydroxymethyl)-3-methyl-1H-pyrazole-4-carboxylic acid**

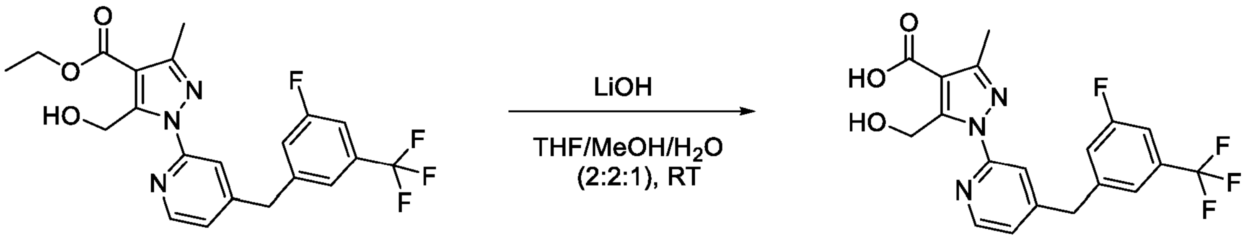


Lithium hydroxide monohydrate (1.07 g, 44.5 mmol) was added to a stirred solution of ethyl 1-(4-(3-fluoro-5-(trifluoromethyl)benzyl)pyridin-2-yl)-5-(hydroxymethyl)-3-methyl-1*H*-pyrazole-4-carboxylate (Intermediate 4, 1.95 g, 4.45 mmol) in THF (10 mL), MeOH (10 mL) and water (5 mL) and the resultant reaction mixture was stirred at RT for 16 h. The solvent was removed *in vacuo.* The residue obtained was dissolved in water (30 mL) and acidified with 2 N HCl to pH ~2 and the aqueous layer extracted with EtOAc (4x50 mL). The combined organic layers were separated, washed with brine (30 mL), dried ($Na_2SO_4$) and the solvent removed *in vacuo* to afford 1-(4-(3-fluoro-5-(trifluoromethyl)benzyl)pyridin-2-yl)-5-(hydroxymethyl)-3-methyl-1*H*-pyrazole-4-carboxylic acid as a white solid (1.81 g, 99%).

**LCMS (Method 1):** m/z 410.0 $(M+H)^+$ (ES+), at 2.49 min.

**$^1$H NMR:** (400 MHz, DMSO-$d_6$) δ: 8.46 (d, *J* = 6.8 Hz, 1H), 7.84 (s, 1H), 7.66-7.55 (m, 3H), 7.41 (d, *J* = 6.8 Hz, 1H), 5.01 (s, 2H), 4.24 (s, 2H), 2.44 (s, 3H). 2 exchangeable protons not observed.

**Preparation of Intermediate 6 (Example 39 of WO2021/181122A1) - 1-(4-(3-fluoro-5-(trifluoromethyl)benzyl)pyridin-2-yl)-5-(hydroxymethyl)-3-methyl-1*H*-pyrazole-4-carboxamide**

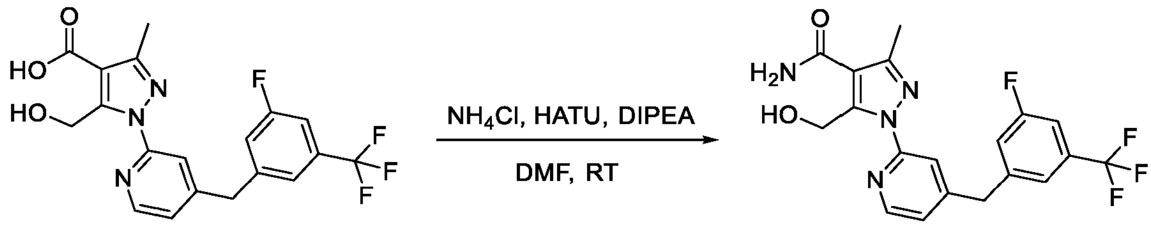


DIPEA (3.06 mL, 17.68 mmol) was added to a stirred solution of 1-(4-(3-fluoro-5-(trifluoromethyl)benzyl) pyridin-2-yl)-5-(hydroxymethyl)-3-methyl-1*H*-pyrazole-4-carboxylic acid (Intermediate 5, 1.81 g, 4.42 mmol) and ammonium chloride (0.354 g, 6.63 mmol) in DMF (50 mL) followed by the addition of HATU (3.36 g, 8.84 mmol) and the resultant reaction mixture was stirred at RT for 16 h. The reaction mixture was partitioned between EtOAc (100 mL) and water (100 mL). The organic layer was separated and the aqueous layer extracted with EtOAc (100 mL). The combined organic layers were washed with brine (50 mL), dried ($Na_2SO_4$) and the solvent removed *in vacuo.* The residue was purified by gradient flash column chromatography eluting with 0-85% EtOAc in pet-ether. The compound was dissolved in MeOH (35 mL) and heated to reflux. The resultant clear solution was allowed to cool to RT and kept undisturbed for 48 h. The recrystalised solid was filtered, rinsed with MeOH (2x10 mL) and dried *in vacuo* to afford 1-(4-(3-fluoro-5-(trifluoromethyl)benzyl)pyridin-2-yl)-5-(hydroxymethyl)-3-methyl-1*H*-pyrazole-4-carboxamide as a white crystalline solid (0.52 g). From the remaining mother liquor, the recrystallisation process was repeated to obtain a further 0.208 g of material. Combined yield 0.728 g, 40%.

**LCMS (Method 2):** m/z 409.0 $(M+H)^+$ (ES+), at 2.08 min

**$^1$H NMR:** (400 MHz, DMSO-$d_6$) δ: 8.44 (d, *J* = 6.8 Hz, 1H), 7.80 (s, 1H), 7.66-7.55 (m, 4H), 7.42-7.37 (m, 2H), 5.63 (t, *J* = 8.4 Hz, 1H), 4.91 (d, *J* = 8.4 Hz, 2H), 4.23 (s, 2H), 2.36 (s, 3H).

## Preparation of Intermediate 7 (Example 40 of WO2021/181122A1) - 1-(4-(3-fluoro-5-(trifluoromethyl)benzyl)pyridin-2-yl)-N-(2-hydroxyethyl)-5-(hydroxymethyl)-3-methyl-1H-pyrazole-4-carboxamide

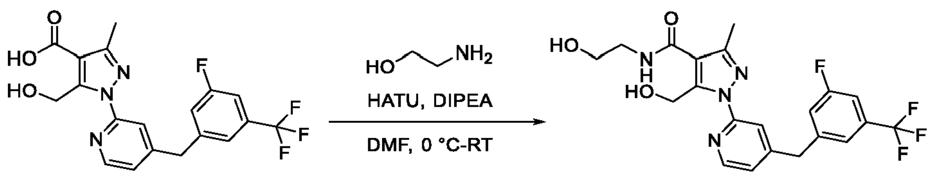


HATU (0.11 g, 0.293 mmol) was added to a stirred solution of 1-(4-(3-fluoro-5-(trifluoromethyl)benzyl)pyridin-2-yl)-5-(hydroxymethyl)-3-methyl-1H-pyrazole-4-carboxylic acid (Intermediate 5, 0.06 g, 0.15 mmol) and 2-aminoethan-1-ol (0.012 g, 0.22 mmol) in DMF (3 mL) at 0 °C followed by the addition of DIPEA (0.038 mg, 0.29 mmol) and the resultant reaction mixture was stirred at RT for 16 h. The reaction mixture was partitioned between EtOAc (30 mL) and water (30 mL). The organic layer was separated and washed with brine (50 mL), dried ($Na_2SO_4$) and the solvent removed in vacuo. The residue was purified by gradient flash column chromatography eluting with 0-50% EtOAc in pet-ether to afford 1-(4-(3-fluoro-5-(trifluoromethyl)benzyl)pyridin-2-yl)-N-(2-hydroxyethyl)-5-(hydroxymethyl)-3-methyl-1H-pyrazole-4-carboxamide as a white solid (18 mg, 27%).

**LCMS (Method 7):** m/z 453.0 (M+H)+ (ES+), at 2.08 min, 95%.

**1H NMR:** (400 MHz, DMSO-d6) δ: 8.44 (d, J = 5.2 Hz, 1H), 8.08 (t, J = 5.6 Hz, 1H), 7.81 (s, 1H), 7.67 (s, 1H), 7.62-7.58 (m, 2H), 7.39 (d, J = 1.2 Hz, 1H), 5.62 (t, J = 6.4 Hz, 1H), 4.89 (d, J = 6.4 Hz, 2H), 4.74 (t, J = 5.6 Hz, 1H), 4.24 (s, 2H), 3.53-3.52 (m, 2H), 3.34-3.30 (m, 2H), 2.34 (s, 3H).

## Preparation of Intermediate 8 - 2-(3-fluoro-5-(trifluoromethyl)benzyl)-4-hydrazineylpyridine

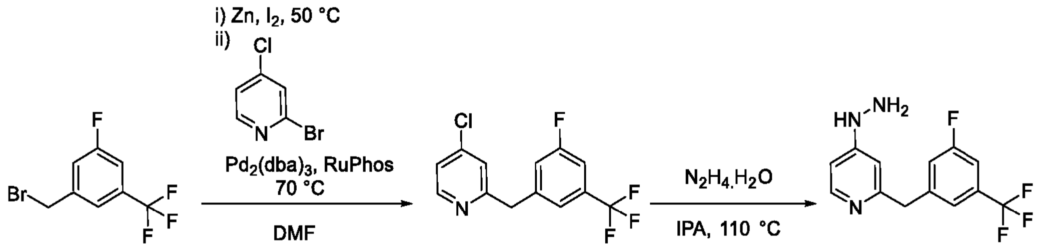


Step 1. A pinch of iodine was added to a stirred solution of activated zinc (35 g, 583 mmol) in DMF (300 mL) and the solution heated at 50 °C for 5 min followed by the addition of 1-(bromomethyl)-3-fluoro-5-(trifluoromethyl)benzene (32 g, 124 mmol) in DMF (50 mL). The reaction mixture was heated at 50 °C for 1 h and then allowed to cool to RT. The residual zinc was allowed to settle and the supernatant pale green DMF layer was transferred *via* cannula to a degassed suspension of 2-bromo-4-chloropyridine (16 g, 83.3 mmol) and RuPhos (2.3 g, 4.99 mmol) in DMF (50 mL) followed by the addition of tris(dibenzylideneacetone)dipalladium(0) (3.8 g, 4.16 mmol). The reaction mixture was heated at 70 °C for 16 h and then filtered through Celite and washed with EtOAc (600 mL). The filtrate was washed with brine (3x300 mL). The organic layer was separated, dried ($Na_2SO_4$) and the solvent removed *in vacuo.* The residue was purified by gradient flash column chromatography eluting with 0-5% EtOAc in pet-ether to afford 4-chloro-2-(3-fluoro-5-(trifluoromethyl)benzyl) pyridine as a yellow semi solid (8 g, 33%).

**LCMS (Method 2):** m/z 290.1 (ES+), at 2.65 min.

**$^1$H NMR:** (400 MHz, DMSO-$d_6$) δ: 8.49 (d, *J* = 5.2 Hz, 1H), 7.83-7.79 (m, 1H), 7.61-7.41 (m, 4H), 4.23 (s, 2H).

Step 2. Hydrazine hydrate (20 g, 415 mmol) was added to a stirred solution of 4-chloro-2-(3-fluoro-5-(trifluoromethyl)benzyl)pyridine (8 g, 27.68 mmol) in IPA (100 mL) in a sealed tube and the reaction mixture was heated at 110 °C for 72 h. The solvent was removed *in vacuo* and the residue partitioned between water (200 mL) and EtOAc (200 mL). The organic layer was separated, washed with brine (200 mL), dried ($Na_2SO_4$) and the solvent removed *in vacuo* to afford 2-(3-fluoro-5-(trifluoromethyl)benzyl)-4-hydrazineylpyridine as a yellow gum (5 g, 63%).

**LCMS (Method 2):** m/z 286.1 (ES+), at 1.19 min.

**$^1$H NMR:** (400 MHz, DMSO-$d_6$) δ: 7.95 (d, *J* = 6.0 Hz, 1H), 7.56-7.21 (m, 4H), 6.59 (d, *J* = 1.6 Hz, 1H), 6.51-6.49 (m, 1H), 4.15 (s, 2H), 3.99 (s, 2H).

### Preparation of Intermediate 9 - 1-(2-(3-fluoro-5-(trifluoromethyl)benzyl)pyridin-4-yl)-5-(hydroxymethyl)-3-methyl-1*H*-pyrazole-4-carboxylic acid

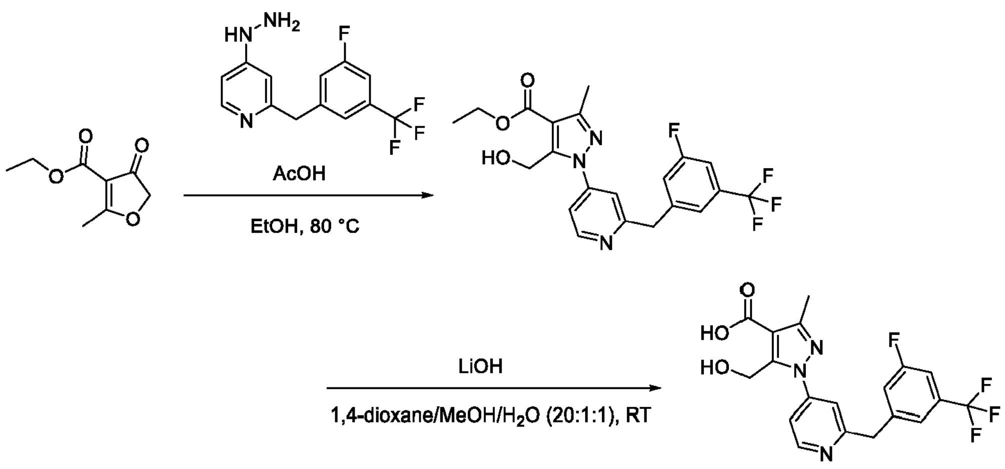


Step 1. 2-(3-fluoro-5-(trifluoromethyl)benzyl)-4-hydrazineylpyridine (Intermediate 8, 600 mg, 2.10 mmol) was added to a stirred solution of ethyl 2-methyl-4-oxo-4,5-dihydrofuran-3-carboxylate (Intermediate 3, 430 mg, 2.52 mmol) in ethanol (20 mL) at RT followed by the addition of catalytic amount of acetic acid (0.120 mL, 2.10 mmol) and the resultant reaction mixture was heated at 80 °C for 16 h. On cooling, the solid which had come out of solution was filtered, rinsed with EtOH (2x10 mL) and dried *in vacuo* to afford ethyl 1-(2-(3-fluoro-5-(trifluoromethyl)benzyl)pyridin-4-yl)-5-(hydroxymethyl)-3-methyl-1*H*-pyrazole-4-carboxylate.

Step 2. LiOH (11.07 mg, 0.462 mmol), water (0.5 mL) and MeOH (0.5 mL) were added to a solution of ethyl

1-(2-(3-fluoro-5-(trifluoromethyl)benzyl)pyridin-4-yl)-5-(hydroxymethyl)-3-methyl-1*H*-pyrazole-4-carboxylate (60 mg, 0.15 mmol) in 1,4-dioxane (20 mL) at RT and the resultant reaction mixture was stirred at RT for 16 h.

After completion of step 2, the title compound (230 mg, 26%) was isolated as a pale yellow solid by removal of the solvent *in vacuo,* acidification with 1.5 N HCl (5 mL) to pH ~ 6 and partitioning between water (10 mL) and EtOAc (15 mL). The organic layer was separated, dried ($Na_2SO_4$) and the solvent removed *in vacuo.*

**LCMS (Method 2):** m/z 410.0 (ES+), at 2.03 min.

**$^1$H NMR:** (400 MHz, DMSO-$d_6$) $\delta$: 8.65 (d, *J* = 7.2 Hz, 1H), 7.80 (s, 1H), 7.72 (d, *J* = 2.4 Hz, 1H), 7.61 (s, 1H), 7.54 (d, *J* = 12.4 Hz, 2H), 4.81 (s, 2H), 4.32 (s, 2H), 3.57 (s, 1H), 2.41 (s, 3H). 1 exchangeable proton not observed.

**Preparation of Intermediate 10 (Example 41 of WO2021/181122A1) - 1-(2-(3-fluoro-5-(trifluoromethyl)benzyl) pyridin-4-yl)-5-(hydroxymethyl)-3-methyl-1H-pyrazole-4-carboxamide**

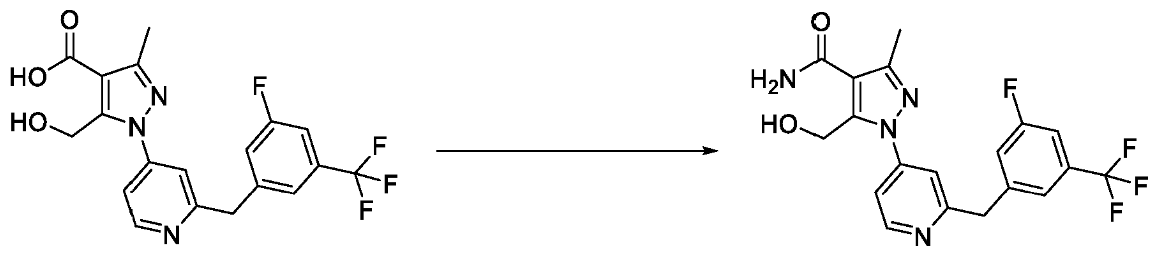

DIPEA (3.06 mL, 17.68 mmol) was added to a stirred solution of 1-(2-(3-fluoro-5-(trifluoromethyl)benzyl) pyridin-4-yl)-5-(hydroxymethyl)-3-methyl-1H-pyrazole-4-carboxylic acid (Intermediate 9, 1.81 g, 4.42 mmol) and ammonium chloride (0.354 g, 6.63 mmol) in DMF (50 mL) followed by the addition of HATU (3.36 g, 8.84 mmol) and the resultant reaction mixture was stirred at RT for 16 h. The reaction mixture was partitioned between EtOAc (100 mL) and water (100 mL). The organic layer was separated and the aqueous layer extracted with EtOAc (100 mL). The combined organic layers were washed with brine (50 mL), dried ($Na_2SO_4$) and the solvent removed *in vacuo.* The residue was purified by gradient flash column chromatography eluting with 0-85% EtOAc in pet-ether. The compound was dissolved in MeOH (35 mL) and heated to reflux. The resultant clear solution was allowed to cool to RT and kept undisturbed for 48 h. The recrystalised solid was filtered, rinsed with MeOH (2x10 mL) and dried *in vacuo* to afford 1-(2-(3-fluoro-5-(trifluoromethyl)benzyl)pyridin-4-yl)-5-(hydroxymethyl)-3-methyl-1H-pyrazole-4-carboxamide.

**LCMS (Method 3):** m/z 409.0 (M+H)+ (ES+), at 1.77 min, 96%.

**$^1$H NMR:** (400 MHz, DMSO-d6) $\delta$: 8.64 (dd, J = 5.4, 1.6 Hz, 1H), 7.74 (s, 1H), 7.63-7.61 (m, 2H), 7.55-7.53 (m, 2H), 7.45 (s, 1H), 7.39 (s, 1H), 5.89 (t, J = 3.6 Hz, 1H), 4.65 (d, J = 5.2 Hz, 2H), 4.31 (s, 2H), 2.37 (s, 3H).

**Preparation of Intermediate 11 (Example 42 of WO2021/181122A1) - 1-(2-(3-fluoro-5-(trifluoromethyl)benzyl) pyridin-4-yl)-N-(2-hydroxyethyl)-5-(hydroxymethyl)-3-methyl-1H-pyrazole-4-carboxamide**

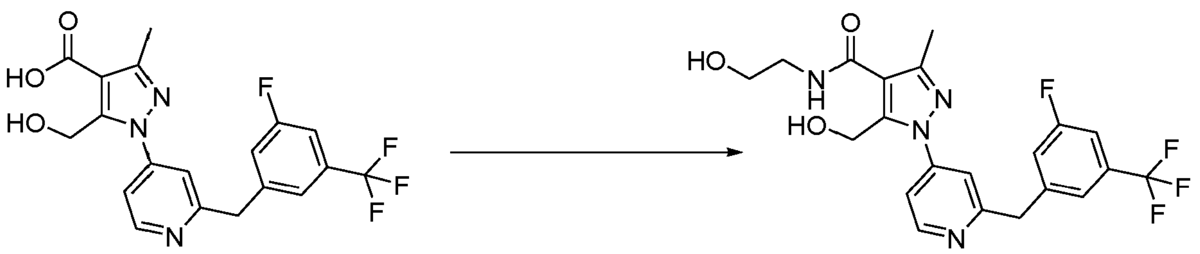

DIPEA (3.06 mL, 17.68 mmol) was added to a stirred solution of 1-(2-(3-fluoro-5-(trifluoromethyl)benzyl) pyridin-4-yl)-5-(hydroxymethyl)-3-methyl-1H-pyrazole-4-carboxylic acid (Intermediate 9, 1.81 g, 4.42 mmol) and ethanolamine (0.354 g, 6.63 mmol) in DMF (50 mL) followed by the addition of HATU (3.36 g, 8.84 mmol) and the resultant reaction mixture was stirred at RT for 16 h. The reaction mixture was partitioned between EtOAc (100 mL) and water (100 mL). The organic layer was separated and the aqueous layer extracted with EtOAc (100 mL). The combined organic layers were washed with brine (50 mL), dried ($Na_2SO_4$) and the solvent removed *in vacuo.* The residue was purified by gradient flash column chromatography eluting with 0-85% EtOAc in pet-ether. The compound was dissolved in MeOH (35 mL) and

heated to reflux. The resultant clear solution was allowed to cool to RT and kept undisturbed for 48 h. The recrystalised solid was filtered, rinsed with MeOH (2x10 mL) and dried *in vacuo* to afford 1-(2-(3-fluoro-5-(trifluoromethyl)benzyl)pyridin-4-yl)-N-(2-hydroxyethyl)-5-(hydroxymethyl)-3-methyl-1H-pyrazole-4-carboxamide.

**LCMS (Method 3):** m/z 453.0 (M+H)+ (ES+), at 1.99 min, 99%.

**$^1$H NMR:** (400 MHz, DMSO-$d_6$) δ: 8.63 (d, *J* = 5.6 Hz, 1H), 7.94 (t, *J* = 5.2 Hz, 1H), 7.74 (s, 1H), 7.64-7.63 (m, 2H), 7.55-7.53 (m, 2H), 5.87 (t, *J* = 4.4 Hz, 1H), 4.75 (t, *J* = 4.4 Hz, 1H), 4.63 (d, *J* = 5.2 Hz, 2H), 4.31 (s, 2H), 3.52 (q, *J* = 5.6 Hz, 2H), 3.37-3.33 (m, 2H), 2.35 (s, 3H).

**Preparation of Intermediate 12 (Example 43 of WO2021/181122A1) - 1-(4-(3-fluoro-5-(trifluoromethyl)benzyl) pyridin-2-yl)-5-(hydroxymethyl)-N,3-dimethyl-1H-pyrazole-4-carboxamide**

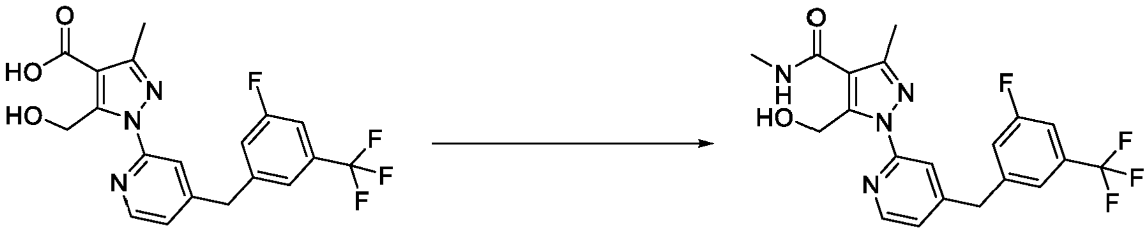

DIPEA (3.06 mL, 17.68 mmol) was added to a stirred solution of 1-(4-(3-fluoro-5-(trifluoromethyl)benzyl) pyridin-2-yl)-5-(hydroxymethyl)-3-methyl-1*H*-pyrazole-4-carboxylic acid (Intermediate 5, 1.81 g, 4.42 mmol) and methylamine (0.354 g, 6.63 mmol) in DMF (50 mL) followed by the addition of HATU (3.36 g, 8.84 mmol) and the resultant reaction mixture was stirred at RT for 16 h. The reaction mixture was partitioned between EtOAc (100 mL) and water (100 mL). The organic layer was separated and the aqueous layer extracted with EtOAc (100 mL). The combined organic layers were washed with brine (50 mL), dried ($Na_2SO_4$) and the solvent removed *in vacuo.* The residue was purified by gradient flash column chromatography eluting with 0-85% EtOAc in pet-ether. The compound was dissolved in MeOH (35 mL) and heated to reflux. The resultant clear solution was allowed to cool to RT and kept undisturbed for 48 h. The recrystalised solid was filtered, rinsed with MeOH (2x10 mL) and dried *in vacuo* to afford 1-(4-(3-fluoro-5-(trifluoromethyl)benzyl)pyridin-2-yl)-5-(hydroxymethyl)-N,3-dimethyl-1H-pyrazole-4-carboxamide.

**LCMS (Method 3):** m/z 422.9 (M+H)+ (ES+), at 2.29 min, 99%.

**$^1$H NMR:** (400 MHz, DMSO-$d_6$) δ: 8.44 (d, *J* = 4.8 Hz, 1H), 7.97 (d, *J* = 4.8 Hz, 1H), 7.81 (s, 1H), 7.67-7.56 (m, 3H), 7.38 (d, *J* = 5.2 Hz, 1H), 5.58 (t, *J* = 6.4 Hz, 1H), 4.87 (d, *J* = 6.4 Hz, 2H), 4.24 (s, 2H), 2.78 (d, *J* = 4.8 Hz, 3H), 2.34 (s, 3H).

**Preparation of Intermediate 13 - 1-(chloromethyl)-3-(difluoromethyl)-5-fluorobenzene**

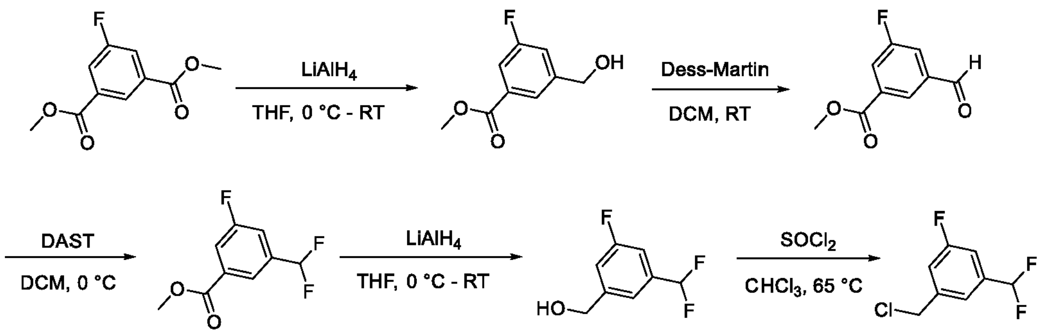


Step 1. $LiAlH_4$ (1.0 M in THF, 7.0 mL, 7.0 mmol) was added to a stirred solution of dimethyl 5-fluoroisophthalate (3 g, 14.1 mmol) in THF (10 mL), at 0 °C and the reaction mixture was stirred at RT for 3h. The reaction mixture was neutralized with 1.5 N HCl (50 mL) to pH ~7, and the reaction mixture was partitioned between water (100 mL) and EtOAc (50 mL). The organic layer was separated, dried ($Na_2SO_4$) and the solvent removed *in vacuo* to afford methyl 3-fluoro-5-(hydroxymethyl)benzoate as a colourless liquid (1.12 g. 43%).

**GCMS (Method 1):** m/z 184.0 (ES+), at 7.34 min.

**$^1$H NMR:** (400 MHz, DMSO-$d_6$) δ: 7.79 (s, 1H), 7.55 (d, *J* = 12.8 Hz, 1H), 7.43 (d, *J* = 12.8 Hz, 1H), 5.49 (t, *J* = 7.6

Hz, 1H), 4.58 (d, *J* = 7.6 Hz, 2H), 3.87 (d, *J* = 2.4 Hz, 3H).

Step 2. Dess-Martin periodinane (2.3 g, 5.54 mmol) was added to a solution of methyl 3-fluoro-5-(hydroxymethyl) benzoate (510 mg, 2.77 mmol) in DCM (10 mL) and the reaction mixture was stirred at RT for 2h. The reaction mixture was filtered and the filtrate was concentrated *in vacuo.* The residue was purified by gradient flash column chromatography eluting with 0-30% EtOAc in hexane to afford methyl 3-fluoro-5-formylbenzoate as a white solid (410 mg, 81%).

**GCMS (Method 1):** m/z 182.0 (ES+), at 6.76 min.

**$^1$H NMR:** (400 MHz, DMSO-$d_6$) δ: 10.08 (d, *J* = 2.4 Hz, 1H), 8.33 (d, *J* = 1.6 Hz, 1H), 8.05-8.04 (m, 2H), 3.92 (s, 3H).

Step 3. DAST (0.44 mL, 3.37 mmol) was added to a solution of methyl 3-fluoro-5-formylbenzoate (410 mg, 2.25 mmol) at 0 °C and the reaction mixture was stirred at RT for 2h. The reaction mixture was neutralized with 10% $NaHCO_3$ (20 mL) to pH ~7, and reaction mixture was partitioned between water (100 mL) and DCM (50 mL). The organic layer was separated, dried ($Na_2SO_4$) and the solvent removed *in vacuo.* The residue was purified by gradient flash column chromatography eluting with 0-30% EtOAc in hexane to afford methyl 3-(difluoromethyl)-5-fluorobenzoate as a colourless liquid (400 mg, 87%).

**GCMS (Method 2):** m/z 204.0 (ES+), at 2.36 min.

**$^1$H NMR:** (400 MHz, DMSO-$d_6$) δ: 7.99 (s, 1H), 7.89 (d, *J* = 11.2 Hz, 1H), 7.80 (d, *J* = 11.2 Hz, 1H), 7.35-6.98 (m, 1H), 3.91 (s, 3H).

Step 4. $LiAlH_4$ (2.0 M in THF, 0.45 mL, 0.90 mmol) was added to a solution of methyl 3-(difluoromethyl)-5-fluorobenzoate (390 mg, 1.81 mmol) in THF (10 mL) at 0 °C and the reaction mixture was stirred at RT for 1h. The reaction mixture was neutralized with 1.5 N HCl (50 mL) to pH ~7 and then partitioned between water (100 mL) and EtOAc (50 mL). The organic layer was separated, dried ($Na_2SO_4$) and the solvent removed *in vacuo* to afford (3-(difluoromethyl)-5-fluorophenyl)methanol as a colourless liquid (230 mg, 72%).

**GCMS (Method 2):** m/z 176.0 (ES+), at 6.36 min.

**$^1$H NMR:** (400 MHz, DMSO-$d_6$) δ: 7.39 (s, 1H), 7.32-7.29 (m, 3H), 5.46 (d, *J* = 6.4 Hz, 1H), 4.57 (t, *J* = 6.4 Hz, 2H).

Step 5. Thionyl chloride (3 mL, 43.2 mmol) was added to a solution of (3-(difluoromethyl)-5-fluorophenyl) methanol (170 mg, 0.96 mmol) in chloroform (10 mL) at RT and the reaction mixture was heated at 65 °C for 12h. The reaction mixture was neutralized with 10% $NaHCO_3$ (20 mL) to pH ~7, then partitioned between water (50 mL) and EtOAc (50 mL). The organic layer was separated, dried ($Na_2SO_4$) and the solvent removed *in vacuo* to afford 1-(chloromethyl)-3-(difluoromethyl)-5-fluorobenzene as a colourless liquid (170 mg, crude). The crude product was used in the next step without further purification.

**GCMS (Method 2):** m/z 193.9 (ES+), at 2.25 min.

**Preparation of Intermediate 14 - 4-(3-(difluoromethyl)-5-fluorobenzyl)-2-hydrazineylpyridine**

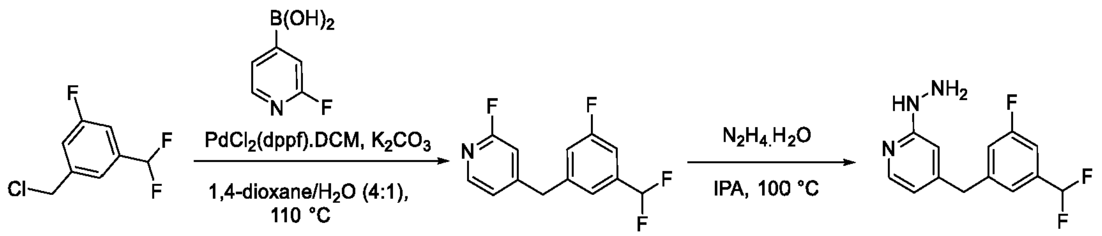


Step 1. $PdCl_2$(dppf).DCM (84 mg, 0.103 mmol) was added to a degassed solution of (2-fluoropyridin-4-yl)boronic acid (145 mg, 1.03 mmol), potassium carbonate (426 mg, 3.09 mmol) and 1-(chloromethyl)-3-(difluoromethyl)-5-fluorobenzene (Intermediate 13, 200 mg, 1.03 mmol) in 1,4-dioxane (8 mL)/water (2 mL) and the resultant reaction mixture heated at 110 °C for 16 h. The reaction mixture was partitioned between water (70 mL) and EtOAc (100 mL). The organic layer was separated, washed with brine (50 mL), dried ($Na_2SO_4$) and the solvent removed *in vacuo.* The residue was purified by gradient flash column chromatography eluting with 0-30% EtOAc in pet-ether to afford 2-fluoro-4-(3-fluoro-5-(difluoromethyl)benzyl)pyridine.

Step 2. Hydrazine hydrate (0.5 mL, 9.77 mmol) was added to a stirred solution of 2-fluoro-4-(3-fluoro-5-(difluoromethyl)benzyl)pyridine in IPA (10 mL) and the resultant reaction mixture was heated at 100 °C for 48 h.

After completion of step 2, the title compound (110 mg, crude) was isolated as a yellow gum by partitioning between EtOAc (10 mL) and water (10 mL). The aqueous layer was extracted with EtOAc (10 mL). The combined organic layers were washed with brine solution (10 mL), dried ($Na_2SO_4$) and the solvent removed *in vacuo.* The crude product was used in the next step without further purification.

**LCMS (Method 3):** m/z 267.9 (ES+), at 3.00 min.

**Preparation of Intermediate 15 - 1-(4-(3-(difluoromethyl)-5-fluorobenzyl)pyridin-2-yl)-5-(hydroxymethyl)-3-methyl-1*H*-pyrazole-4-carboxylic acid**

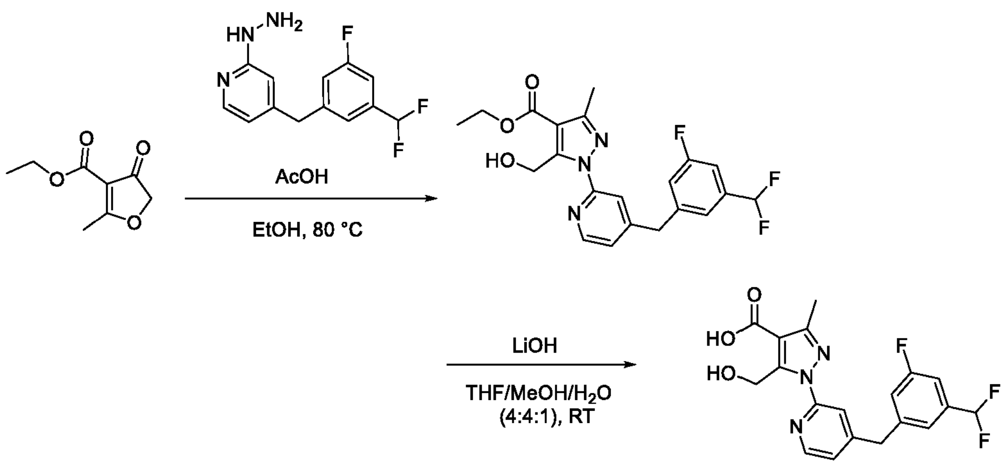


Step 1. 4-(3-(difluoromethyl)-5-fluorobenzyl)-2-hydrazineylpyridine (Intermediate 14, 1.0 g, 3.74 mmol) was added to a stirred solution of ethyl 2-methyl-4-oxo-4,5-dihydrofuran-3-carboxylate (Intermediate 3), 0.638 g, 3.74 mmol) in ethanol (30 mL) at RT followed by the addition of catalytic amount of acetic acid (0.021 mL, 0.374 mmol) and the resultant reaction mixture was heated at 80 °C for 16 h. On cooling, the solid which had come out of solution was filtered, rinsed with EtOH (2x10 mL) and dried *in vacuo* to afford ethyl 1-(4-(3-fluoro-5-(difluoromethyl)benzyl)pyridin-2-yl)-5-(hydroxymethyl)-3-methyl-1*H*-pyrazole-4-carboxylate.
Step 2. Lithium hydroxide monohydrate (0.756 g, 31.6 mmol) was added to a stirred solution of ethyl 1-(4-(3-fluoro-5-(difluoromethyl)benzyl)pyridin-2-yl)-5-(hydroxymethyl)-3-methyl-1*H*-pyrazole-4-carboxylate (1.95 g, 4.45 mmol) in THF (20 mL), MeOH (20 mL) and water (5 mL) and the resultant reaction mixture was stirred at RT for 16 h.
After completion of step 2, the title compound was isolated as a white solid by removal of the solvent *in vacuo,* dissolution in water (50 mL) and acidification with 2 N HCl to pH ~ 2. The aqueous layer was extracted with EtOAc (4x50 mL). The combined organic layers were washed with brine (50 mL), dried ($Na_2SO_4$) and the solvent removed *in vacuo.*
**LCMS (Method 2):** m/z 392.0 (ES+), at 2.48 min.
**$^1$H NMR:** (300 MHz, DMSO-$d_6$) δ: 8.40 (d, *J* = 5.1 Hz, 1H), 7.74 (s, 1H), 7.45-7.41 (m, 2H), 7.32-7.30 (m, 2H), 7.03 (t, *J* = 55.5 Hz, 1H), 4.92 (d, *J* = 8.1 Hz, 2H), 4.18 (s, 2H), 2.38 (s, 3H). 2 exchangeable protons not observed.

**Preparation of Intermediate 16 (Example 44 of WO2021/181122A1) - 1-(4-(3-(difluoromethyl)-5-fluorobenzyl) pyridin-2-yl)-5-(hydroxymethyl)-3-methyl-1H-pyrazole-4-carboxamide**

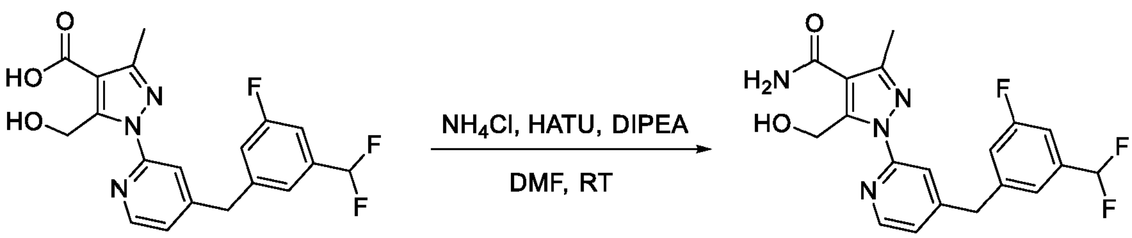


DIPEA (3.06 mL, 17.68 mmol) was added to a stirred solution of 1-(4-(3-(difluoromethyl)-5-fluorobenzyl) pyridin-2-yl)-5-(hydroxymethyl)-3-methyl-1*H*-pyrazole-4-carboxylic acid (Intermediate 15, 1.81 g, 4.42 mmol) and ammonium chloride (0.354 g, 6.63 mmol) in DMF (50 mL) followed by the addition of HATU (3.36 g, 8.84 mmol) and the resultant reaction mixture was stirred at RT for 16 h. The reaction mixture was partitioned between EtOAc (100 mL) and water (100 mL). The organic layer was separated and the aqueous layer extracted with EtOAc (100 mL). The combined organic layers were washed with brine (50 mL), dried ($Na_2SO_4$) and the solvent removed *in vacuo.* The residue was

purified by gradient flash column chromatography eluting with 0-85% EtOAc in pet-ether. The compound was dissolved in MeOH (35 mL) and heated to reflux. The resultant clear solution was allowed to cool to RT and kept undisturbed for 48 h. The recrystalised solid was filtered, rinsed with MeOH (2x10 mL) and dried *in vacuo* to afford 1-(4-(3-(difluoromethyl)-5-fluorobenzyl)pyridin-2-yl)-5-(hydroxymethyl)-3-methyl-1H-pyrazole-4-carboxamide.

**LCMS (Method 2):** m/z 391.0 (M+H)+ (ES+), at 1.88 min, 97%.

**$^1$H NMR:** (400 MHz, DMSO-$d_6$) δ: 8.44 (d, *J* = 5.2 Hz, 1H), 7.78 (s, 1H), 7.53-7.50 (m, 1H), 7.46-7.43 (m, 3H), 7.37-7.31 (m, 2H), 7.03 (t, *J* = 55.2 Hz, 1H), 5.64 (t, *J* = 6.4 Hz, 1H), 4.91 (d, *J* = 6.4 Hz, 2H), 4.20 (s, 2H), 2.37 (s, 3H).

**PREPARATION OF EXAMPLES**

**Preparation of Example 1 - (4-carbomyl-1-(4-(3-fluoro-5-(trifluoromethyl)benzyl)pyridine-2-yl)-3-methyl-1 *H*-pyrazol-5-yl)methyl acetate**

**Scheme 2**

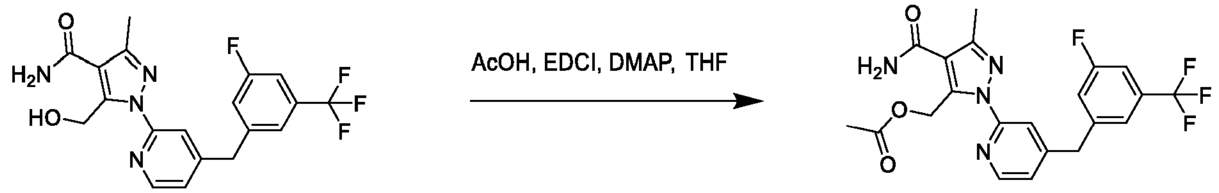


1-[4-[[3-fluoro-5-(trifluoromethyl)phenyl]methyl]-2-pyridyl]-5-(hydroxymethyl)-3-methylpyrazole-4-carboxamide (Intermediate 6, 100 mg, 0.24 mmol), acetic acid (0.03 mL, 0.49 mmol), 1-(3-dimethylaminopropyl)-3-ethylcarbodiimide hydrochloride (93.89 mg, 0.49 mmol), and DMAP (59.84 mg, 0.49 mmol) were added to THF (10 mL). The reaction mixture was stirred at room temperature overnight. The reaction mixture was partitioned between ethyl acetate (40 ml) and water (40 ml). The organic layer was dried ($MgSO_4$) and the solvent evaporated off in vacuo. The residue was purified by flash column chromatography (normal phase, [5.9 x 2.0 cm (10 g)], Biotage® SNAP KP-Sil - 50 μm irregular silica, 45 mL per min, [gradient 0% to 100% Ethyl Acetate in Iso-hexane], 0-100 psi, [residue loaded in a small volume of DCM]) to afford 2 (4-carbamoyl-1-(4-(3-fluoro-5-(trifluoromethyl)benzyl)pyridin-2-yl)-3-methyl-1 H-pyrazol-5-yl)methyl dihydrogen phosphate as a colourless solid (79 mg, 72%).

[4-carbamoyl-2-[4-[[3-fluoro-5-(trifluoromethyl)phenyl]methyl]-2-pyridyl]-5-methyl-pyrazol-3-yl]methyl acetate (53 mg, 0.1200 mmol) was dissolved in a minimum amount of near boiling ethyl acetate. The solution was allowed to cool to room temperature and left overnight. Solvent evaporated- solid remnant looks crystalline. Dried in vacuum oven for 3 hours at 40°C to give 2 (4-carbamoyl-1-(4-(3-fluoro-5-(trifluoromethyl)benzyl)pyridin-2-yl)-3-methyl-1 *H*-pyrazol-5-yl) methyl dihydrogen phosphate as a colourless, crystalline solid (41 mg, 77%).

**LCMS (Method 5):** m/z 451.2. $(M+H)^+$ (ES+), at 4.11 min

**1H NMR:** (400 MHz, $CDCl_3$) d: 8.30 (d, *J* = 6.8 Hz, 1H), 7.64 (s, 1H), 7.04-6.97 (m, 4H), 7.21-7.16 (m, 2H), 5.75 (s, 2H), 4.04 (s, 2H), 2.44 (s, 3H), 1.90 (s, 3H)

**Preparation of Example 2 - (4-carbamoyl-1-(4-(3-fluoro-5-(trifluoromethyl)benzyl)pyridine-2-yl)-3-methyl-1 H-pyrazol-5-yl) dihydrogen phosphate**

## Scheme 3

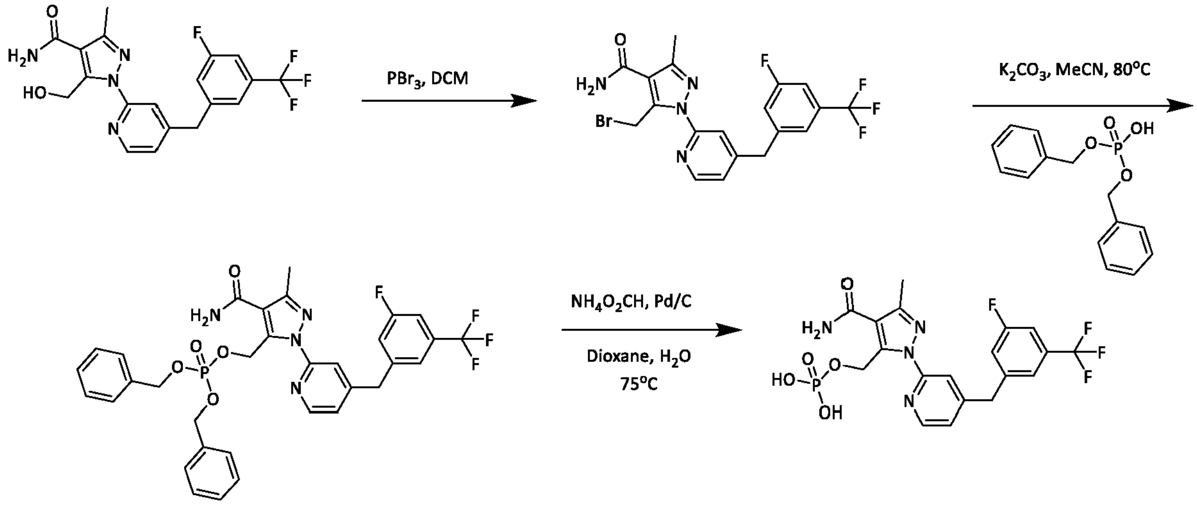


**Step 1** - **5-(bromomethyl)-1-(4-(3-fluoro-5-(trifluoromethyl)benzyl)pyridin-2-yl)-3-methyl-1 *H*-pyrazole-4-carboxamide**

1-[4-[[3-fluoro-5-(trifluoromethyl)phenyl]methyl]-2-pyridyl]-5-(hydroxymethyl)-3-methylpyrazole-4-carboxamide (Intermediate 6, 2500 mg, 6.12 mmol) was added to DCM (20 mL). phosphorus tribromide (1.74 mL, 18.37 mmol) was added to the reaction mixture and stirred at room temperature overnight. The reaction mixture was partitioned between ethyl acetate (40 ml) and water (40 ml). The organic layer was dried ($MgSO_4$) and solvent evaporated in vacuo. The residue was triturated with ether: isohexane (1:1, 25 ml) and filtered off the solid, washed the solid twice with ether:isohexane (1:1, 2x25 ml) to afford 5-(bromomethyl)-1-(4-(3-fluoro-5-(trifluoromethyl)benzyl)pyridin-2-yl)-3-methyl-1 *H*-pyrazole-4-carboxamide as a colourless solid (2200 mg, 76%).

**LCMS (Method 4):** m/z 471.2 ($^{79}$Br), 473.2 ($^{81}$Br) $(M+H)^+$ (ES+), at 1.66 min.

**1H NMR:** (400 MHz, DMSO-$d_6$) d: 8.40-8.32 (m, 1H), 7.82 (s, 1H), 7.56-7.27 (m, 6H), 5.30 (s, 2H), 4.15 (s, 2H), 2.34 (s, 3H).

**Step 2** - **dibenzyl ((4-carbamoyl-1-(4-(3-fluoro-5-(trifluoromethyl)benzyl)pyridin-2-yl)-3-methyl-1H-pyrazol-5-yl) methyl) phosphate**

5-(bromomethyl)-1-[4-[[3-fluoro-5-(trifluoromethyl)phenyl]methyl]-2-pyridyl]-3-methylpyrazole-4-carboxamide (2100 mg, 4.46 mmol), dibenzyl hydrogen phosphate (1487.88 mg, 5.35 mmol), and potassium carbonate (1231.79 mg, 8.91 mmol) were added to MeCN (10 mL). The reaction mixture was heated at 80°C for 2 hours. The reaction mixture was partitioned between ethyl acetate (50 ml) and water (50 ml). Dried the organics ($MgSO_4$) and evaporated in vacuo. The residue was purified by flash column chromatography (normal phase, [5.9 x 2.0 cm (10 g)], Biotage® SNAP KP-Sil - 50 μm irregular silica, 50 mL per min, [gradient 0% to 5% MeOH in DCM], 0-100 psi / 7 bar, [residue loaded in a small volume of DCM]) to afford dibenzyl ((4-carbamoyl-1-(4-(3-fluoro-5-(trifluoromethyl)benzyl)pyridin-2-yl)-3-methyl-1H-pyrazol-5-yl) methyl) phosphate as a colourless solid (2700 mg, 87%).

**LCMS (Method 4):** m/z 669.2 $(M+H)^+$ (ES+), at 1.81 min.

**1H NMR:** (400 MHz, DMSO-$d_6$) d: 8.22 (d, J= 6.8 Hz, 1H), 7.53 (s, 1H), 7.24-7.10 (m, 12H), 6.96-6.93 (m, 1H), 6.88-6.86 (m, 1H), 5.74 (d, *J* = 7.9 Hz, 2H), 4.79 (s, 4H), 4.05 (s, 2H), 2.41 (s, 3H). 2 exchangeable protons not seen.

**Step 3** - **Example 2 (4-carbamoyl-1-(4-(3-fluoro-5-(trifluoromethyl)benzyl)pyridine-2-yl)-3-methyl-1 H-pyrazol-5-yl) dihydrogen phosphate**

Dibenzyl [4-carbamoyl-2-[4-[[3-fluoro-5-(trifluoromethyl)phenyl]methyl]-2-pyridyl]-5-methyl-pyrazol-3-yl] methyl phosphate (2700.00 mg, 4.04 mmol) was dissolved in IPA (200 mL) and water (10 mL). Ammonium formate (2546.66 mg, 40.38 mmol) was added to the solution, the solution was flushed with nitrogen. Palladium on activated charcoal (49.46 mg, 0.40 mmol) was added to the reaction mixture under an atmosphere of nitrogen and heated at 75°C for 2 h. The palladium catalyst was filtered off using a Biotage Isolute Celite 545 cartridge (2.5 g/ 25 mL). The solvent in the mother liquors was evaporated off in vacuo. The residue was purified by reverse phase chiral chromatography- using the following method Instruments: Gilson Semi Preparative HPLC System - 321 Pump/171 Diode Array Detector/GX-271

Liquid Handler; Column: Phenomenex Luna PFP 5 micron 30 x 100 mm; Gradient 12.5 min, solvent A is acetonitrile, solvent B is 0.1% aqueous solution of trifluoroacetic acid, gradient 30% of A to 60% of A over 12.5 minutes to afford (4-carbamoyl-1-(4-(3-fluoro-5-(trifluoromethyl)benzyl)pyridine-2-yl)-3-methyl-1 H-pyrazol-5-yl) dihydrogen phosphate as a colourless solid (310 mg, 16%).

**LCMS (Method 6):** m/z 489.0 $(M+H)^+$ (ES+), at 2.64 mins

**1H NMR:** (400 MHz, DMSO-$d_6$) δ: 8.40 (d, *J* = 6.8 Hz, 1H), 7.76 (s, 2H), 7.66-7.55 (m, 3H), 7.46 (s, 1H), 7.33 (s, 1H), 5.52 (d, *J* = 7.9 Hz, 2H), 4.23 (s, 2H), 2.34 (s, 3H). 2 exchangeable protons not observed.

Further examples prepared by the above procedures are detailed in Table 2. The skilled person can modify the methods where appropriate, for example through use of protecting groups where appropriate.

**Table 2: Examples table**

| Example | Name | Intermediate/procedure |
|---|---|---|
| 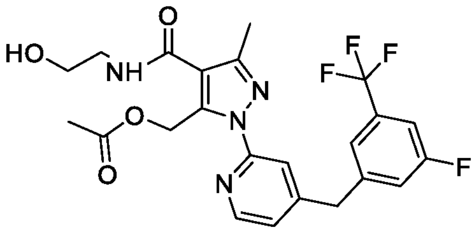 3 | (1-(4-(3-fluoro-5-(trifluoromethyl)benzyl)pyridin-2-yl)-4-((2-hydroxyethyl) carbamoyl)-3-methyl-1H-pyrazol-5-yl)methyl acetate | Intermediate 7 using the method of Example 1 |
| 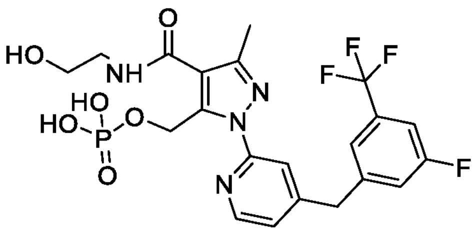 4 | (1-(4-(3-fluoro-5-(trifluoromethyl)benzyl)pyridin-2-yl)-4-((2-hydroxyethyl) carbamoyl)-3-methyl-1H-pyrazol-5-yl)methyl dihydrogen phosphate | Intermediate 7 using the method of Example 2 |
| 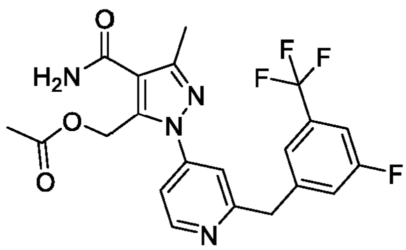 5 | (4-carbamoyl-1-(2-(3-fluoro-5-(trifluoromethyl)benzyl)pyridin-4-yl)-3-methyl-1H-pyrazol-5-yl)methyl acetate | Intermediate 10 using the method of Example 1 |
| 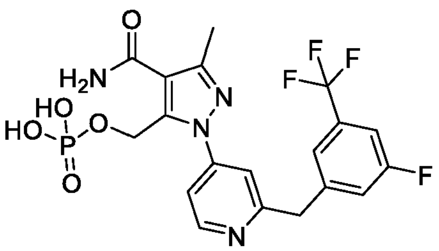 6 | (4-carbamoyl-1-(2-(3-fluoro-5-(trifluoromethyl)benzyl)pyridin-4-yl)-3-methyl-1H-pyrazol-5-yl)methyl dihydrogen phosphate | Intermediate 10 using the method of Example 2 |

(continued)

| Example | Name | Intermediate/procedure |
|---|---|---|
| 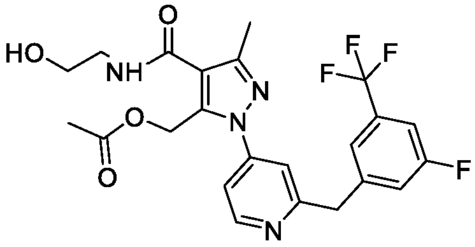 7 | (1-(2-(3-fluoro-5-(trifluoromethyl)benzyl)pyridin-4-yl)-4-((2-hydroxyethyl) carbamoyl)-3-methyl-1H-pyrazol-5-yl)methyl acetate | Intermediate 11 using the method of Example 1 |
| 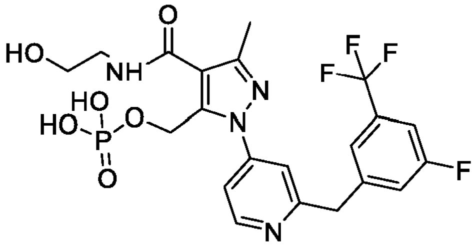 8 | (1-(2-(3-fluoro-5-(trifluoromethyl)benzyl)pyridin-4-yl)-4-((2-hydroxyethyl) carbamoyl)-3-methyl-1H-pyrazol-5-yl)methyl dihydrogen phosphate | Intermediate 11 using the method of Example 2 |
| 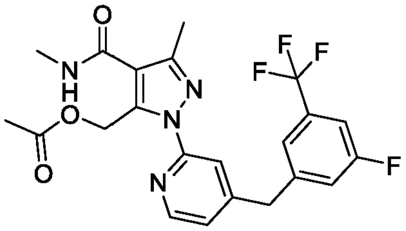 9 | (1-(4-(3-fluoro-5-(trifluoromethyl)benzyl)pyridin-2-yl)-3-methyl-4-(methylcarbamoyl)-1H-pyrazol-5-yl)methyl acetate | Intermediate 12 using the method of Example 1 |
| 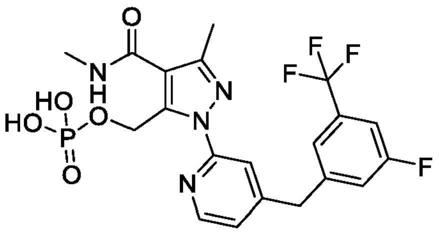 10 | (1-(4-(3-fluoro-5-(trifluoromethyl)benzyl)pyridin-2-yl)-3-methyl-4-(methylcarbamoyl)-1H-pyrazol-5-yl)methyl dihydrogen phosphate | Intermediate 12 using the method of Example 2 |
| 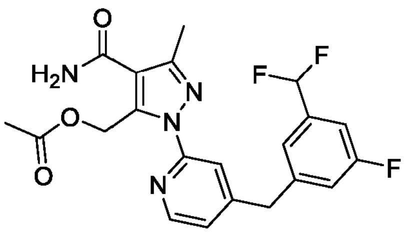 11 | (4-carbamoyl-1-(4-(3-(difluoromethyl)-5-fluorobenzyl)pyridin-2-yl)-3-methyl-1H-pyrazol-5-yl)methyl acetate | Intermediate 16 using the method of Example 1 |

(continued)

| Example | Name | Intermediate/procedure |
|---|---|---|
| 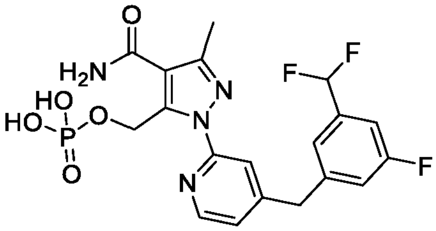 12 | (4-carbamoyl-1-(4-(3-(difluoromethyl)-5-fluorobenzyl)pyridin-2-yl)-3-methyl-1H-pyrazol-5-yl)methyl dihydrogen phosphate | Intermediate 16 using the method of Example 2 |

## BIOLOGICAL ACTIVITY

### GPR52 Agonist Functional cAMP Assay

HEKf suspension cells were infected for 24 h with 0.1% v/v human GPR52 expressing BacMam virus, a modified baculovirus designed for mammalian gene expression. Following BacMam infection, cells were pelleted by centrifugation (335 g, 5 min), resuspended in cell freezing medium (Sigma) and frozen at -150 °C until required. On experiment day, 25 nL GPR52 compound dilutions, prepared in DMSO, were stamped onto proxiplates (PerkinElmer) by a LabCyte ECHO acoustic dispenser. Frozen cells were thawed and resuspended in assay stimulation buffer (Cisbio) containing 0.5mM 3-iso-butyl-1-methylxanthine (IBMX, Sigma) to achieve a density of 2000 cells per well. 10 μl cells were added to assay plates using a Multidrop Combi Reagent Dispenser (ThermoFisher) before centrifugation (1 min). Cells were incubated with compounds at 37 °C for 30 min prior to addition of cAMP detection reagents (HiRange cAMP kit, Cisbio) which were prepared according to the manufacturer's instructions. Plates were shaken for 1 h at room temperature before reading on a PHERAstar FS plate reader (BMG Labtech) using standard HTRF settings. HTRF ratios were obtained by dividing the acceptor emissions (665 nm) by the donor emissions (620 nm) and multiplying by 10,000. Data were normalised to DMSO (0%) and maximal 3-(2-(3-chloro-5-fluorobenzyl)benzo[b]thiophen-7-yl)-N-(2-methoxyethyl)benzamide (compound 7m in *J. Med. Chem.,* 2014, 57, 5226) responses (100%) and fit to a 4-parameter logistical fit to generate agonist $pEC_{50}$s and maximal responses which are presented in **Table 3** below. Further pharmacokinetic data for Intermediate 6 is provided in Applicant's earlier application PCT/GB2021/050638 (see Example 39).

**Table 3 - GPR52 $pEC_{50}$ data**

| Compound | $pEC_{50}$ average | $E_{max}$ (%) |
|---|---|---|
| Intermediate 6 (Example 39 of WO2021/181122A1) | 8.2 | 101 |
| Intermediate 7 (Example 40 of WO2021/181122A1) | 8.6 | 101 |
| Intermediate 10 (Example 41 of WO2021/181122A1) | 7.5 | 98 |
| Intermediate 11 (Example 42 of WO2021/181122A1) | 7.5 | 100 |
| Intermediate 12 (Example 43 of WO2021/181122A1) | 8.0 | 101 |
| Intermediate 16 (Example 44 of WO2021/181122A1) | 8.4 | 101 |

### Caffeine-induced locomotor activity in rat of Intermediate 6 (Example 39 of WO2021/181122A1)

Caffeine, a non-selective adenosine receptor antagonist, is a psychostimulant which increases rodent locomotor activity principally via blockade of $A_{2A}$ receptors (*Br. J. Pharmacol.,* 2000, *129,* 1465). These receptors are densely expressed on the terminals of GABAergic striatopallidal neurons in the indirect pathway of the basal ganglia, in which dopamine D2 receptors are co-expressed (*J. Comp. Neural*., 1998, *401,* 163; *J. Comp. Neurol.,* 2001, *431,* 331). Tonic activation of $A_{2A}$ receptors decreases the affinity of D2 receptors to dopamine and antagonism of $A_{2A}$ receptors facilitates dopaminergic signalling (Curr. Pharm. Des., 2008, 14, 1468). A number of antipsychotic agents have been shown to block hyperlocomotion induced by caffeine (Pharmacol. Biochem. Behav., 1994, 47, 89; Naunyn-Schmiedeberg's Arch. Pharmacol., 2016, 389, 11).

Male Sprague-Dawley rats (200-250 g) were housed in groups with a 12 h light/dark cycle (lights on at 07.00), at an ambient temperature of 21 ± 2 °C and with standard pelleted diet and water *ad libitum.* Testing was carried out in the

light phase. On the day of the experiment, animals were habituated to the locomotor cages for a 60-minute period. Subsequently, they were dosed with vehicle or Intermediate 6 (Example 39 of WO2021/181122A1) (0.1, 0.3, 1 and 3 mg/kg) by the oral route and returned to the appropriate locomotor cage. Intermediate 6 (Example 39 of WO2021/181122A1) was formulated in a vehicle of 10% DMAC, 10% solutol (Kolliphor HS15) and 80% water (v/v/v). Sixty minutes later, animals were dosed with vehicle (saline) or caffeine (15 mg/kg) by the subcutaneous route. Locomotor activity was assessed for a 2 h period after caffeine treatment. Data are back-transformed means, adjusted for differences between treatment groups in activity during the 30 minutes prior to treatment with test compound or vehicle (n = 10-12). Analysis was by general linear model with treatment, cohort and rack as factors. SEMs were calculated from the residuals of the statistical model. Intermediate 6 (Example 39 of WO2021/181122A1) was compared to caffeine by Williams' test.

As shown in Figure 1, treatment with Intermediate 6 (Example 39 of WO2021/181122A1) caused a dose-dependent reduction of the caffeine-induced hyperlocomotor response, reaching statistical significance at 1 and 3 mg/kg.

**Pharmacokinetic profiling of Example 2**

Systemic exposure of Example 2 and Intermediate 6 (Example 39 of Applicant's earlier application PCT/GB2021/050638 (WO2021/181122A1)) were assessed in male Sprague-Dawley rats and male Beagle dogs following oral (per os, PO) administration of Example 2. Plasma concentration-time data (mean values ± standard deviation) are detailed in **Tables 4** and **5.** Plasma concentrations of Example 2 were below the limit of quantification (1 ng/mL) at all time points in both rat and dog, demonstrating no detectable oral bioavailability. Conversely, mean maximal plasma concentrations (Cmax) of Intermediate 6 were 240 ng/mL and 1079 ng/mL in rat and dog respectively, demonstrating conversion of Example 2 to, and bioavailability of, Intermediate 6 *in vivo.*

**Methods:**

Three male Sprague-Dawley rats (weighing 269-270 g) and three male Beagle dogs (weighing 7-10 kg), were administered a single dose of Example 2 via PO route, using the dose and vehicle specified in Table #. Following dosing, blood samples were taken at several time points (pre-dose, 5 min, 15 min, 30 min, 1 h, 2 h, 4 h, 8 h, 12 h and 24 h in both species, plus additional samples at 32 h and 48 h in dog) via serial bleeding of jugular vein (rat) or cephalic vein (dog), and centrifuged to separate plasma for quantitative bioanalysis of Example 2 and Intermediate 6 by LC-MS/MS.

**Table 4: Plasma concentration-time data after PO dose of Example 2 in Beagle dogs**

| | | | | Example 2 | | Intermediate 6 | |
|---|---|---|---|---|---|---|---|
| Dose (mg/kg) | Dose route | Dosing vehicle | Sampling time (h) | Mean conc (ng/mL) | SD | Mean conc (ng/mL) | SD |
| 3 | PO | 1% methylcellulose (400 cP) in water | Pre-dose | BQL | - | BQL | |
| *BQL = Below quantifiable limit of 1 ng/mL* | | | 0.083 | BQL | - | 6 | 5 |
| | | | 0.25 | BQL | - | 589 | 427 |
| | | | 0.5 | BQL | - | 1079 | 244 |
| | | | 1 | BQL | - | 1010 | 130 |
| | | | 2 | BQL | - | 737 | 183 |
| | | | 4 | BQL | - | 437 | 91 |
| | | | 8 | BQL | - | 199 | 89 |
| | | | 12 | BQL | - | 126 | 54 |
| | | | 24 | BQL | - | 175 | 96 |
| | | | 32 | BQL | - | 84 | 49 |
| | | | 48 | BQL | - | 50 | 58 |

**Table 5: Plasma concentration-time data after PO dose of Example 2 in Sprague-Dawley rats**

| | | | | Example 2 | | Intermediate 6 | |
|---|---|---|---|---|---|---|---|
| Dose (mg/kg) | Dose route | Dosing vehicle | Sampling time (h) | Mean conc (ng/mL) | SD | Mean conc (ng/mL) | SD |
| 3 | PO | 1% methylcellulose (400 cP) in water | Pre-dose | BQL | - | BQL | |
| *BQL = Below quantifiable limit of 1 ng/mL* | | | 0.083 | BQL | - | 8 | 6 |
| | | | 0.25 | BQL | - | 107 | 65 |
| | | | 0.5 | BQL | - | 206 | 116 |
| | | | 1 | BQL | - | 240 | 70 |
| | | | 2 | BQL | - | 239 | 37 |
| | | | 4 | BQL | - | 221 | 45 |
| | | | 8 | BQL | - | 68 | 19 |
| | | | 12 | BQL | - | 32 | 19 |
| | | | 24 | BQL | - | BQL | 58 |

## Solubility of Example 2

This experiment was designed to evaluate the thermodynamic solubility of Example 2 in various biorelevant media (Blank Fasted State Simulated Intestinal Fluid (FaSSIF) pH 6.5, FaSSIF pH 6.5 and simulated gastric fluid (SGF) without enzyme pH 1.2).

A 10 mM stock solution of Example 2 in dimethyl sulfoxide (DMSO) was prepared. From the 10 mM stock solution, a 1 mM intermediate solution of Example 2 in DMSO was prepared. A working solution of 1 μM of Example 2 was prepared by diluting the intermediate solution in mobile phase solution (methanol: 2 mM ammonium acetate containing suitable internal standard - carbamazepine). The working solution was serially diluted in mobile phase solution up to 8 linearity points to prepare standard solutions for plotting a calibration curve. The area for each standard solution was analyzed using LCMS/MS. The normalized area values were plotted vs. concentration to achieve a calibration equation.

Example 2 (1 mg) was added to 1 mL of each desired biorelevant media (SGF without enzyme pH 1.2, Blank FaSSIF pH 6.5 and FaSSIF pH 6.5) to achieve a theoretical concentration equivalent to 1 mg/mL.

The resulting solutions were then kept on RotoSpin (shaker) at 50 rpm for 4 hours at room temperature (25 °C). After the incubation period, the content was filtered through a 0.45 μm PVDF (hydrophilic) syringe filter and the filtrate was taken for quantification by LCMS/MS. The filtrate was diluted in mobile phase and subsequently, the area under the curve (AUC) was ascertained for diluted sample using LCMS/MS. From the AUC of test sample the corresponding concentration was calculated using 6 to 8 point linearity/calibration curve.

Solubilities of Example 2 in various biorelevant media are in **Table 6.**

**Table 6: Solubility of Example 2 in biorelevant media**

| Media | Solubility (μM) |
|---|---|
| Blank FaSSIF, pH 6.5 | 1887.5 |
| FaSSIF, pH 6.5 | 1831 |
| SGF, pH 1.2 | 13.069 |

## Pharmacokinetic profiling of Intermediate 6 (Example 39 of WO2021/181122A1)

The pharmacokinetic profiles of Intermediate 6 (Example 39 of WO2021/181122A1) were assessed in male Sprague-Dawley rats via intravenous (IV) delivery. Pharmacokinetic data (mean values ± standard deviation) for Intermediate 6 (Example 39 of WO2021/181122A1) are detailed in **Table 7.**

**Methods:**

For pharmacokinetic analysis, groups of three male Sprague-Dawley rats, ranging in weight between 200 and 230 g, were administered a single dose of Intermediate 6 (Example 39 of WO2021/181122A1) via IV, using the dose, dose volume and vehicle specified in **Table 7.** Following dosing, blood samples were taken at several time points (pre-dose, 2 min, 5 min, 15 min, 30 min, 1 h, 3 h, 6 h, 12 h and 24 h for IV) via serial tail vein bleeds, and centrifuged to separate plasma for analysis by LC-MS/MS. WinNonlin v8.2 statistics software (Pharsight Corporation, California, USA) was used to generate pharmacokinetic parameters using non-compartmental analysis.

**Brain penetration of Intermediate 6 (Example 39 of WO2021/181122A1)**

Plasma and brain exposure were evaluated to assess the brain penetration of Intermediate 6 (Example 39 of WO2021/181122A1), following IV administration. Unbound brain-to-plasma ratio ($K_{p,uu}$) was calculated, as detailed in **Table 7,** following experimental determination of binding in rat plasma and brain homogenate.

**Methods:**

For brain penetration assessment, male Sprague-Dawley rats (n=3) were administered a single 1 mg/kg dose (formulated in 10% DMAC + 10% Solutol HS15 + 80% saline) via the IV route. After 10 min post-dose, animals were sacrificed and brains extracted, homogenised with 2 volumes (w/v) of 50 mM sodium phosphate buffer (pH 7.4), and analysed by LC-MS/MS. Blood samples were removed at the same time point via tail vein bleed, centrifuged and the plasma analysed by LC-MS/MS.

To permit calculation of unbound brain-to-plasma ratio ($K_{p,uu}$), test compound binding in rat plasma and brain homogenate was performed, using Rapid Equilibrium Dialysis (RED). Test compound prepared in DMSO (1 μM final, 0.2% DMSO) was added to (i) undiluted male Sprague Dawley rat plasma and (ii) rat brain tissue homogenised with 2 volumes (w/v) of sodium phosphate buffer (pH 7.4), and dialysed against phosphate buffer for 5 h at 37 °C. After incubation, the contents of each plasma/brain and buffer compartment were removed and mixed with equal volumes of control dialysed buffer or plasma/brain to maintain matrix similarity for analysis. Proteins were then precipitated by the addition of acetonitrile containing an analytical internal standard (allowing ratio of test compound versus internal standard to be derived), centrifuged and the supernatant removed for analysis by LC-MS/MS. Fraction unbound ($F_u$) in plasma and brain was calculated using the following formula, then used to correct total plasma and brain concentrations to derive the $K_{p,uu}$:

Fraction bound = (Total plasma or brain ratio) - (Total buffer ratio)/Total plasma or brain ratio Fraction unbound ($F_u$, brain or plasma) = 1 - Fraction bound

For correction of dilution in brain binding assay:

$$\text{Undiluted } F_u\text{,brain} = (1 / \text{dilution factor}) / ((1 / F_u \text{ diluted})) - 1) + (1 / \text{dilution factor})$$

Where dilution factor = 4

**Table 7: Plasma pharmacokinetic and brain penetration data after IV dose of Intermediate 6 (Example 39 of WO2021/181122A1) in Sprague-Dawley rats**

| | **Rat IV pharmacokinetics (n=3)** | | | |
|---|---|---|---|---|
| | Dose (mg/kg) | Dose volume (mL/kg) | Dosing vehicle | Clearance (mL/min/kg) |
| Intermediate 6 | 1 | 5 | 10 % DMAC + 10 % Solutol HS15 + 80 % saline | 8.8 ± 1.1 |
| | **Rat IV brain penetration, 10 min (n=3)** | | | |
| | Dose (mg/kg) | Dose volume (mL/kg) | Dosing vehicle | $K_{p,uu}$ |
| Intermediate 6 | 1 | 5 | 10 % DMAC + 10 % Solutol HS15 + saline | 0.42 ± 0.08 |

## Brief description of the Figures

Figure 1: The effect of acute treatment with Intermediate 6 (Example 39 of WO2021/181122A1) (0.1, 0.3, 1 and 3 mg/kg, PO) on caffeine-induced hyperlocomotor activity. Significant differences vs caffeine are represented as * $p<0.05$, ** $p<0.01$, *** $p<0.001$.

## Claims

1. A compound of Formula (1a) or (1b):

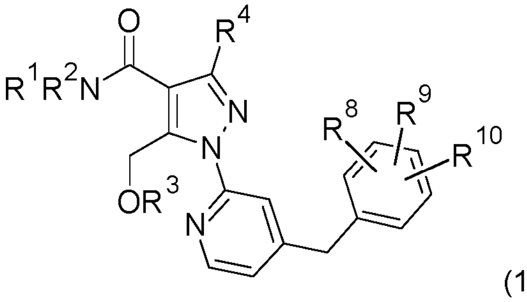
(1a);

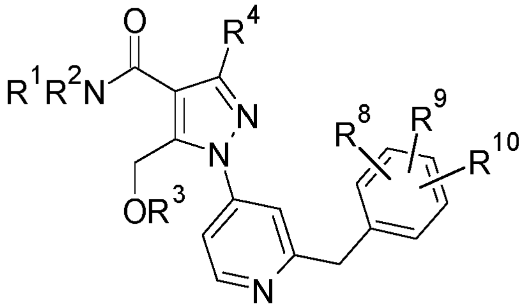
(1b);

or a salt thereof, wherein;

$R^1$ and $R^2$ are independently H or $C_{1-3}$ alkyl optionally substituted with OH or 1 to 6 fluorine atoms; or $R^1$ and $R^2$ are joined to form a 4, 5, 6 or 7-membered ring which is optionally substituted with OH or 1 to 6 fluorine atoms;
$R^3$ is $-P(O)OR^6OR^7$ or $-COR^5$;
$R^4$ is H, CN, halo, or $C_{1-3}$ alkyl optionally substituted with OH or 1 to 6 fluorine atoms;
$R^5$ is $C_{1-6}$ alkyl optionally substituted with 1 to 6 fluorine atoms, 3- to 6-membered heterocyclyl, or $C_{3-6}$ cycloalkyl;
$R^6$ and $R^7$ are independently H, $C_{1-6}$ alkyl optionally substituted with 1 to 6 fluorine atoms, 3-to 6-membered heterocyclyl or $C_{3-6}$ cycloalkyl; or $R^6$ and $R^7$ are joined to form a 5- or 6-membered ring; and
$R^8$, $R^9$ and $R^{10}$ are independently H, CN, halo, or $C_{1-3}$ alkyl optionally substituted with 1 to 6 fluorine atoms.

2. The compound according to claim 1, or a salt thereof, wherein $R^3$ is $-P(O)OR^6OR^7$ or $-COR^5$;

wherein $R^5$ is $C_{1-6}$ alkyl optionally substituted with 1 to 6 fluorine atoms; and
$R^6$ and $R^7$ are independently H or $C_{1-6}$ alkyl optionally substituted with 1 to 6 fluorine atoms, preferably wherein $R^6$ and $R^7$ are both H.

3. The compound according to claim 1 or claim 2, wherein $R^1$ and $R^2$ are both H, or a salt thereof.

4. The compound according to any one of claims 1 to 3, wherein $R^4$ is H or methyl, or a salt thereof.

5. The compound according to any one of claims 1 to 4, wherein $R^8$, $R^9$ and $R^{10}$ are independently selected from H, F, $CHF_2$ and $CF_3$, or a salt thereof.

6. The compound according to any one of claims 1 to 5, wherein the group:

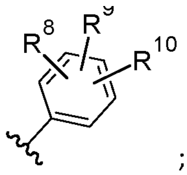
;

is:

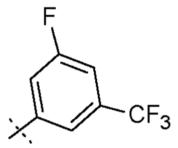 ; or 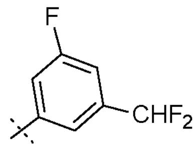 ;

or a salt thereof.

7. The compound according to claim 1 or claim 2, which is a compound of formula (3):

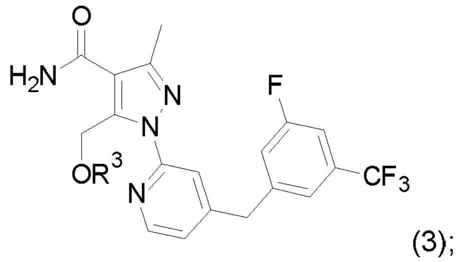

(3);

or a salt thereof.

8. The compound according to any one of claims 1 to 7, wherein $R^3$ is $-PO_3H_2$, or a salt thereof.

9. The compound according to any one of claims 1 to 7, wherein $R^3$ is -COMe, or a salt thereof.

10. The compound according to claim 1, which is selected from the group consisting of:

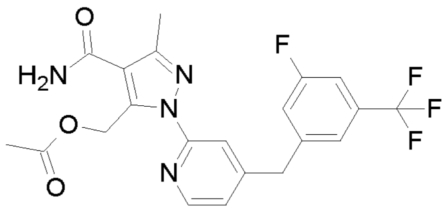 ; 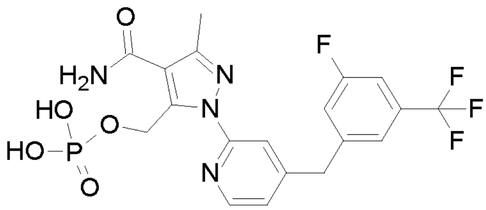 ;

or a salt thereof.

11. A pharmaceutical composition comprising a compound or salt as defined in any one of claims 1 to 10 and a pharmaceutically acceptable excipient.

12. The compound or salt according to any one of claims 1 to 10 or composition according to claim 11 for use in medicine.

13. The compound or salt according to any one of claims 1 to 10 or composition according to claim 11 for use in the treatment of psychiatric disorders; neuropsychiatric disorders; neurodegenerative disorders; psychotic disorders; cognitive disorders; neurocognitive disorders; extrapyramidal disorders; movement disorders; motor disorders; hyperkinetic movement disorders; catatonia; mood disorders; depressive disorders; anxiety disorders; obsessive-compulsive disorder (OCD); autism spectrum disorders; depressive disorders; hypothalamic disorders; pituitary disorders; prolactin-related disorders; trauma- or stressor-related disorders; disruptive, impulse-control or conduct disorders; sleep-wake disorders; substance-related disorders; addictive disorders; behavioral disorders; hypofrontality; abnormalities in the tuberoinfundibular, mesolimbic, mesocortical, or nigrostriatal pathway; decreased activity in the striatum; cortical dysfunction; neurocognitive dysfunction or conditions or symptoms related thereto.

14. The compound, salt or composition for use according to claim 13, wherein the disorder or symptom is selected from schizophrenia, positive symptoms of schizophrenia, negative symptoms of schizophrenia, cognitive symptoms of schizophrenia, depression, attention-deficit hyperactivity disorder (ADHD), generalised anxiety disorder, obsessive-compulsive disorder (OCD), panic disorder, bipolar disorder, addiction/impulse-control disorders, autism spectrum

disorders, psychosis, anhedonia, agitation, Alzheimer's disease, Parkinson's disease, Huntington's disease, vascular dementia, Lewy body disease, frontotemporal dementia, Tourette's syndrome, hyperprolactinemia, pituitary adenoma, prolactinoma, craniopharyngioma, Cushing's disease, diabetes insipidus, non-functioning tumours, obesity, posttraumatic stress disorder (PTSD), akathisia and associated movements, athetosis, ataxia, ballismus, hemiballismus, chorea, choreoathetosis, dyskinesia, tardive dyskinesia, neuroleptic-induced dyskinesia, myoclonus, mirror movement disorder, paroxysmal kinesigenic dyskinesia, restless legs syndrome, spasms, stereotypic movement disorder, sterotypy, Tic disorder, tremor, Wilson's disease, schizotypal personality disorder, delusional disorder, brief psychotic disorder, schizophreniform disorder, schizoaffective disorder, substance- or medication-induced psychotic disorder, delusions, hallucinations, disorganized thinking, grossly disorganized or abnormal motor behavior, catatonia, major depressive disorder, bipolar I disorder, bipolar II disorder, cyclothymic disorder, substance- or medication-induced bipolar and related disorders, bipolar and related disorders due to another medical condition, separation anxiety disorder, selective mutism, specific phobia, social anxiety disorder, panic disorder, agoraphobia, generalized anxiety disorder, substance- or medication-induced anxiety disorder, anxiety disorders due to another medical condition, delirium, major neurocognitive disorder, minor neurocognitive disorder, amnesia, dementia, developmental coordination disorder, stereotypic movement disorder, a post-stroke effect, dentatorubral-pallidoluysian atrophy, diminished emotional expression, avolition, alogia and asociality.

**15.** The compound, salt or composition for use according to claim 13, wherein the disorder or symptom is selected from schizophrenia, positive symptoms of schizophrenia, negative symptoms of schizophrenia, cognitive symptoms of schizophrenia, depression, attentiondeficit hyperactivity disorder (ADHD), generalised anxiety disorder, obsessive-compulsive disorder (OCD), panic disorder, bipolar disorder, addiction/impulse-control disorders, autism spectrum disorders, psychosis, neurocognitive disorder, delirium, anhedonia, agitation, Alzheimer's disease, Parkinson's disease, Huntington's disease, vascular dementia, Lewy body disease, frontotemporal dementia, Tourette's syndrome, hyperprolactinemia, obesity, and posttraumatic stress disorder (PTSD).

**Patentansprüche**

**1.** Verbindung der Formel (1a) oder (1b):

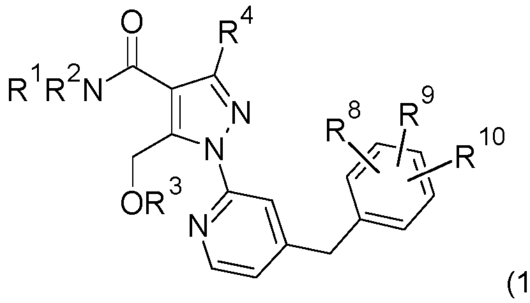

(1a);

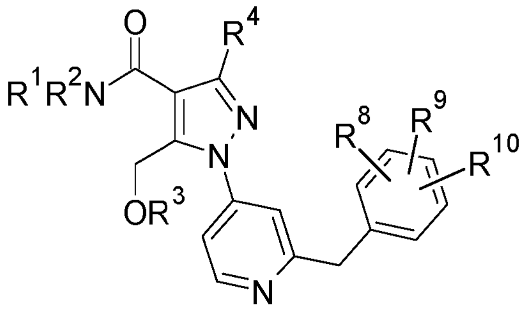

(1b);

oder ein Salz davon, wobei;

$R^1$ und $R^2$ unabhängig H oder $C_{1-3}$-Alkyl sind, optional substituiert mit OH oder 1 bis 6 Fluoratomen; oder $R^1$ und $R^2$ verbunden sind, um einen 4-, 5-, 6- oder 7-gliedrigen Ring zu bilden, der optional mit OH oder 1 bis 6 Fluoratomen substituiert ist;
$R^3$ -P(O)$OR^6OR^7$ oder -$COR^5$ ist;
$R^4$ H, CN, Halogen oder $C_{1-3}$-Alkyl ist, optional substituiert mit OH oder 1 bis 6 Fluoratomen; $R^5$ $C_{1-6}$-Alkyl ist, optional substituiert mit 1 bis 6 Fluoratomen, 3- bis 6-gliedriges Heterocyclyl oder $C_{3-6}$ -Cycloalkyl;
$R^6$ und $R^7$ unabhängig H, $C_{1-6}$-Alkyl, optional substituiert mit 1 bis 6 Fluoratomen, 3- bis 6-gliedriges Heterocyclyl oder $C_{3-6}$-Cycloalkyl sind; oder $R^6$ und $R^7$ verbunden sind, um einen 5- oder 6-gliedrigen Ring zu bilden; und
$R^8$, $R^9$ und $R^{10}$ unabhängig H, CN, Halogen oder $C_{1-3}$-Alkyl, optional substituiert mit 1 bis 6 Fluoratomen, sind.

**2.** Verbindung nach Anspruch 1, oder ein Salz davon, wobei $R^3$ -P(O)$OR^6OR^7$ oder - $COR^5$ ist;

wobei $R^5$ $C_{1-6}$-Alkyl ist, optional substituiert mit 1 bis 6 Fluoratomen; und
$R^6$ und $R^7$ unabhängig H oder $C_{1-6}$-Alkyl, optional substituiert mit 1 bis 6 Fluoratomen, sind, vorzugsweise, wobei

$R^6$ und $R^7$ beide H sind.

3. Verbindung nach Anspruch 1 oder Anspruch 2, wobei $R^1$ und $R^2$ beide H oder ein Salz davon sind.

4. Verbindung nach einem der Ansprüche 1 bis 3, wobei $R^4$ H oder Methyl oder ein Salz davon ist.

5. Verbindung nach einem der Ansprüche 1 bis 4, wobei $R^8$, $R^9$ und $R^{10}$ unabhängig ausgewählt sind aus H, F, $CHF_2$ und $CF_3$ oder einem Salz davon.

6. Zusammensetzung nach einem der Ansprüche 1 bis 5, wobei die Gruppe:

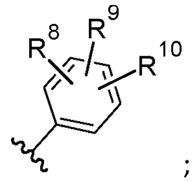

;

ist:

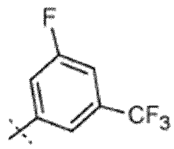

; oder

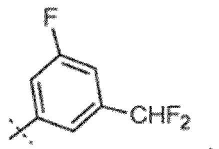

;

oder ein Salz davon.

7. Verbindung nach Anspruch 1 oder Anspruch 2, die eine Verbindung der Formel (3) ist:

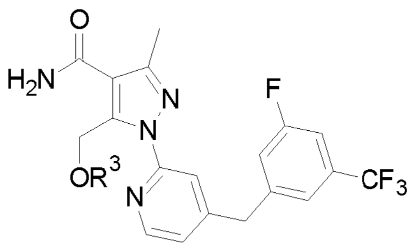

(3);

oder ein Salz davon.

8. Verbindung nach einem der Ansprüche 1 bis 7, wobei $R^3$ $-PO_3H_2$ oder ein Salz davon ist.

9. Verbindung nach einem der Ansprüche 1 bis 7, wobei $R^3$ -COMe oder ein Salz davon ist.

10. Verbindung nach Anspruch 1, die aus der Gruppe ausgewählt ist, bestehend aus:

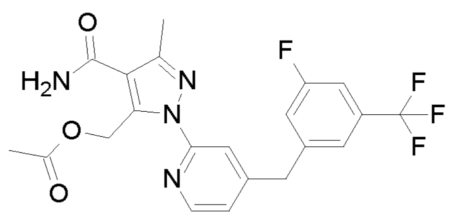

;

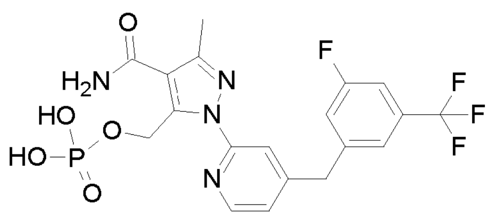

;

oder ein Salz davon.

11. Pharmazeutische Zusammensetzung, umfassend eine Verbindung oder ein Salz, wie in einem der Ansprüche 1 bis 10 definiert, und einen pharmazeutisch annehmbaren Hilfsstoff.

12. Verbindung oder Salz nach einem der Ansprüche 1 bis 10 oder Zusammensetzung nach Anspruch 11 zur Verwendung in der Medizin.

13. Verbindung oder Salz nach einem der Ansprüche 1 bis 10 oder Zusammensetzung nach Anspruch 11 zur Verwendung bei der Behandlung von psychiatrischen Störungen; neuropsychiatrischen Störungen; neurodegenerativen Störungen; psychotischen Störungen; kognitiven Störungen; neurokognitiven Störungen; extrapyramidalen Störungen; Bewegungsstörungen; motorischen Störungen; hyperkinetischen Bewegungsstörungen; Katatonie; affektiven Störungen; depressiven Störungen; Angststörungen; Zwangsstörungen (OCD); Autismus-Spektrum-Störungen; depressiven Störungen; Hypothalamusstörungen; Hypophysenstörungen; Prolaktin-bedingten Störungen; trauma- oder stressbedingten Störungen; störenden Impulskontroll- oder Verhaltensstörungen; Schlaf-Wach-Störungen; substanzbedingten Störungen; Suchtstörungen; Verhaltensstörungen; Hypofrontalität; Anomalien im tuberoinfundibulären, mesolimbischem, mesokortikalem oder nigrostriatalem Pfad; verminderter Aktivität im Striatum; kortikaler Dysfunktion; neurokognitiver Dysfunktion oder damit verbundenen Zuständen oder Symptomen.

14. Verbindung, Salz oder Zusammensetzung zur Verwendung nach Anspruch 13, wobei die Störung oder das Symptom ausgewählt ist aus Schizophrenie, positiven Symptomen der Schizophrenie, negativen Symptomen der Schizophrenie, kognitiven Symptomen der Schizophrenie, Depression, Aufmerksamkeitsdefizit-Hyperaktivitätsstörung (ADHS), generalisierter Angststörung, Zwangsstörung (OCD), Panikstörung, bipolarer Störung, Sucht-/Impulskontrollstörungen, Autismus-Spektrum-Störungen, Psychose, Anhedonie, Unruhe, Alzheimer-Krankheit, Parkinson-Krankheit, Huntington-Krankheit, vaskulärer Demenz, Lewy-Körperchen-Krankheit, frontotemporaler Demenz, Tourette-Syndrom, Hyperprolaktinämie, Hypophysenadenom, Prolaktinom, Kraniopharyngeom, Morbus Cushing, Diabetes insipidus, nicht-funktionierenden Tumoren, Adipositas, posttraumatischer Belastungsstörung (PTBS), Akathisie und damit verbundene Bewegungen, Athetose, Ataxie, Ballismus, Hemiballismus, Chorea, Choreoathetose, Dyskinesie, tardiver Dyskinesie, neuroleptisch induzierter Dyskinesie, Myoklonus, Spiegelbewegungsstörung, paroxysmaler kinesigener Dyskinesie, Restless-Legs-Syndrom, Spasmen, stereotyper Bewegungsstörung, Stereotypie, Tic-Störung, Tremor, Morbus Wilson, schizotypischer Persönlichkeitsstörung, wahnhafter Störung, kurzer psychotischer Störung, schizophreniformer Störung, schizoaffektiver Störung, substanzoder medikamenteninduzierter psychotischer Störung, Wahnvorstellungen, Halluzinationen, desorganisiertem Denken, stark desorganisiertem oder abnormalem motorischem Verhalten, Katatonie, schwerer depressiver Störung, bipolarer I-Störung, bipolarer II-Störung, zyklothymischer Störung, substanz- oder medikamenteninduzierter bipolarer und verwandter Störungen, bipolarer und verwandter Störungen aufgrund einer anderen medizinischen Erkrankung, Trennungsangststörung, selektivem Mutismus, spezifischer Phobie, sozialer Angststörung, Panikstörung, Agoraphobie, generalisierter Angststörung, durch Substanzen oder Medikamente induzierter Angststörung, Angststörungen aufgrund einer anderen Erkrankung, Delirium, schwerer neurokognitiver Störung, leichter neurokognitiver Störung, Amnesie, Demenz, Entwicklungskoordinationsstörung, stereotyper Bewegungsstörung, Folgeerscheinungen nach Schlaganfall, dentatorubral-pallidoluysianischer Atrophie, verminderter emotionaler Ausdrucksfähigkeit, Antriebslosigkeit, Alogie und Asozialität.

15. Verbindung, Salz oder Zusammensetzung zur Verwendung nach Anspruch 13, wobei die Störung oder das Symptom aus Schizophrenie, positiven Symptomen der Schizophrenie, negativen Symptomen der Schizophrenie, kognitiven Symptomen der Schizophrenie, Depression, Aufmerksamkeitsdefizit-Hyperaktivitätsstörung (ADHS), generalisierter Angststörung, Zwangsstörung (OCD), Panikstörung, bipolarer Störung, Sucht-/Impulskontrollstörungen, Autismus-Spektrum-Störungen, Psychose, neurokognitiver Störung, Delirium, Anhedonie, Unruhe, Alzheimer-Krankheit, Parkinson-Krankheit, Huntington-Krankheit, vaskulärer Demenz, Lewy-Körperchen-Krankheit, frontotemporaler Demenz, Tourette-Syndrom, Hyperprolaktinämie, Adipositas und posttraumatischer Belastungsstörung (PTBS) ausgewählt ist.

## Revendications

1. Composé de Formule (1a) ou (1b) :

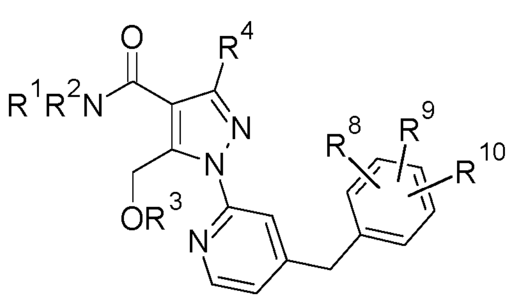 (1a); 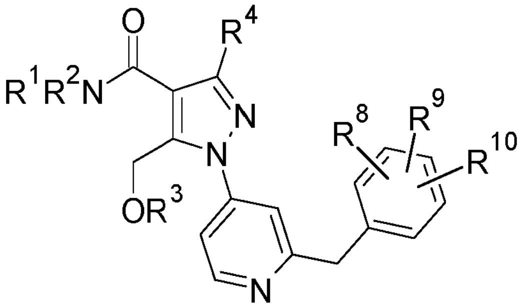 (1b);

ou un sel de celui-ci, dans lequel :

$R^1$ et $R^2$ sont indépendamment un H ou un groupe alkyle en $C_{1-3}$ facultativement substitué par un OH ou 1 à 6 atomes de fluor ; ou $R^1$ et $R^2$ sont liés pour former un cycle à 4, 5, 6 ou 7 chaînons qui est facultativement substitué par un OH ou 1 à 6 atomes de fluor ;
$R^3$ est $-P(O)OR^6OR^7$ ou $-COR^5$ ;
$R^4$ est un H, un CN, un halo, ou un alkyle en $C_{1-3}$ facultativement substitué par un OH ou 1 à 6 atomes de fluor ;
$R^5$ est un alkyle en $C_{1-6}$ facultativement substitué par 1 à 6 atomes de fluor, un hétérocyclyle à 3 à 6 chaînons, ou un cycloalkyle en $C_{3-6}$ ;
$R^6$ et $R^7$ sont indépendamment un H, un alkyle en $C_{1-6}$ facultativement substitué par 1 à 6 atomes de fluor, un hétérocyclyle à 3 à 6 chaînons ou un cycloalkyle en $C_{3-6}$ ; ou $R^6$ et $R^7$ sont liés pour former un cycle à 5 ou 6 chaînons ; et
$R^8$, $R^9$ et $R^{10}$ sont indépendamment un H, un CN, un halo, ou un alkyle en $C_{1-3}$ facultativement substitué par 1 à 6 atomes de fluor.

**2.** Composé selon la revendication 1, ou un sel de celui-ci, dans lequel $R^3$ est - $P(O)^6OR^7$ ou $-COR^5$ ;
dans lequel $R^5$ est un alkyle en $C_{1-6}$ facultativement substitué par 1 à 6 atomes de fluor ; et $R^6$ et $R^7$ sont indépendamment un H ou un alkyle en $C_{1-6}$ facultativement substitué par 1 à 6 atomes de fluor, de préférence dans lequel $R^6$ et $R^7$ sont tous les deux un H.

**3.** Composé selon la revendication 1 ou la revendication 2, dans lequel $R^1$ et $R^2$ sont tous les deux un H, ou un sel de celui-ci.

**4.** Composé selon l'une quelconque des revendications 1 à 3, dans lequel $R^4$ est un H ou un méthyle, ou un sel de celui-ci.

**5.** Composé selon l'une quelconque des revendications 1 à 4, dans lequel $R^8$, $R^9$ et $R^{10}$ sont indépendamment sélectionnés parmi H, F, $CHF_2$ et $CF_3$, ou un sel de celui-ci.

**6.** Composé selon l'une quelconque des revendications 1 à 5, dans lequel le groupe :

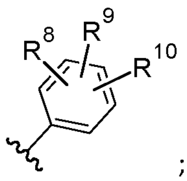 ;

est :

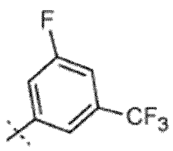 ; ou 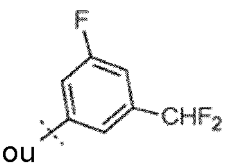 ;

ou un sel de celui-ci.

**7.** Composé selon la revendication 1 ou la revendication 2, qui est un composé de Formule (3) :

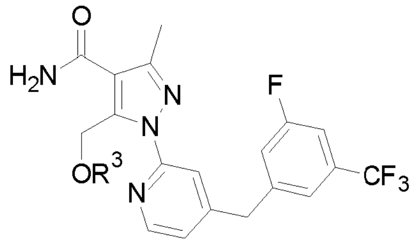

(3);

ou un sel de celui-ci.

**8.** Composé selon l'une quelconque des revendications 1 à 7, dans lequel $R^3$ est - $PO_3H_2$, ou un sel de celui-ci.

**9.** Composé selon l'une quelconque des revendications 1 à 7, dans lequel $R^3$ est - COMe, ou un sel de celui-ci.

**10.** Composé selon la revendication 1, qui est sélectionné dans le groupe consistant en :

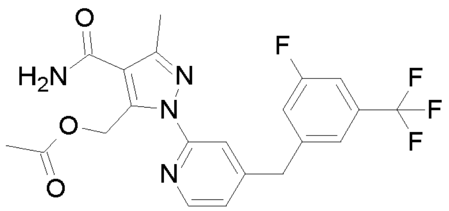

;

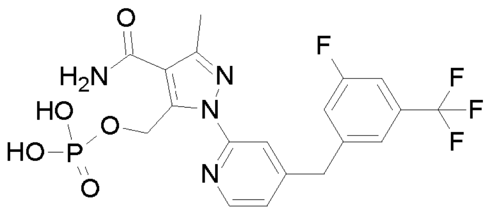

;

ou un sel de celui-ci.

**11.** Composition pharmaceutique comprenant un composé ou un sel tel que défini dans l'une quelconque des revendications 1 à 10 et excipient pharmaceutiquement acceptable.

**12.** Composé ou sel selon l'une quelconque des revendications 1 à 10 ou composition selon la revendication 11 pour une utilisation en médecine.

**13.** Composé ou sel selon l'une quelconque des revendications 1 à 10 ou composition selon la revendication 11 pour une utilisation dans le traitement de troubles psychiatriques ; de troubles neuropsychiatriques ; de troubles neurodégénératifs ; de troubles psychotiques ; de troubles cognitifs ; de troubles neurocognitifs ; de troubles extrapyramidaux ; de troubles du mouvement ; de troubles moteurs ; de troubles hyperkinétiques du mouvement ; de la catatonie ; de troubles de l'humeur ; de troubles dépressifs ; de troubles de l'anxiété ; de troubles obsessionnels compulsifs (TOC) ; de troubles du spectre de l'autisme ; de troubles dépressifs ; de troubles hypothalamiques ; de troubles hypophysaires ; de troubles liés à la prolactine ; de troubles liés à un traumatisme ou à un facteur de stress ; de troubles perturbateurs, du contrôle des impulsions ou de la conduite ; de troubles du cycle sommeil-éveil ; de troubles liés à une substance ; de troubles de la dépendance ; de troubles du comportement ; de l'hypofrontalité ; d'anomalies dans la voie tubéro-infundibulaire, mésolimbique, mésocorticale, ou nigrostriatale ; d'une diminution d'activité dans le striatum ; d'un dysfonctionnement cortical ; d'un dysfonctionnement neurocognitif ou d'états ou de symptômes qui y sont apparentés.

**14.** Composition, sel ou composition pour une utilisation selon la revendication 13, dans laquelle ou lequel le trouble ou le symptôme est sélectionné parmi schizophrénie, symptômes positifs de la schizophrénie, symptômes négatifs de la schizophrénie, symptômes cognitifs de la schizophrénie, dépression, trouble déficitaire de l'attention avec hyperactivité (TDAH), trouble anxieux généralisé, trouble obsessionnel compulsif (TOC), trouble panique, trouble bipolaire, troubles de la dépendance / du contrôle des impulsions, troubles du spectre de l'autisme, psychose, anhédonie, agitation, maladie d'Alzheimer, maladie de Parkinson, maladie de Huntington, démence vasculaire, maladie à corps de Lewy, démence fronto-temporale, syndrome de Tourette, hyperprolactinémie, adénome hypophysaire, prolactinome, craniopharyngiome, maladie de Cushing, diabète insipide, tumeurs non fonctionnelles, obésité, trouble de stress post-traumatique (TSPT), akathisie et mouvements associés, athétose, ataxie, ballisme, hémiballisme, chorée, choréoathétose, dyskinésie, dyskinésie tardive, dyskinésie induite par les neuroleptiques, myoclonie, syndrome des mouvements en miroir, dyskinésie kinésigénique paroxystique, syndrome des jambes sans repos, spasmes, trouble des mouvements stéréotypés, stéréotypie, tics, tremblements, maladie de Wilson, trouble de la personnalité schizotypique, trouble délirant, trouble psychotique bref, trouble schizophréniforme, trouble schizoaffectif, trouble psychotique induit par une substance ou un médicament, délires, hallucinations, pensée désorganisée, comportement moteur extrêmement désorganisé ou anormal, catatonie, trouble dépressif majeur, trouble bipolaire I, trouble bipolaire II, trouble cyclothymique, troubles bipolaires et apparentés induits par une substance ou un médicament, troubles bipolaires et apparentés dus à un autre état médical, trouble d'anxiété de séparation, mutisme sélectif, phobie spécifique, trouble d'anxiété sociale, trouble panique, agoraphobie, trouble d'anxiété généralisée, trouble d'anxiété induit par une substance ou un médicament, troubles d'anxiété dus à un autre état médical, délirium, trouble neurocognitif majeur, trouble neurocognitif mineur, amnésie, démence, trouble développemental de la coordination, trouble des mouvements stéréotypés, effet post-AVC, atrophie dentato-rubro-pallido-luysienne, diminution de l'expression émotionnelle, avolition, alogie et asocialité.

**15.** Composition, sel ou composition pour une utilisation selon la revendication 13, dans laquelle ou lequel le trouble ou le symptôme est sélectionné parmi schizophrénie, symptômes positifs de la schizophrénie, symptômes négatifs de la schizophrénie, symptômes cognitifs de la schizophrénie, dépression, trouble déficitaire de l'attention avec hyperactivité (TDAH), trouble anxieux généralisé, trouble obsessionnel compulsif (TOC), trouble panique, trouble bipolaire, troubles de la dépendance / du contrôle des impulsions, troubles du spectre de l'autisme, psychose, trouble neurocognitif, délirium, anhédonie, agitation, maladie d'Alzheimer, maladie de Parkinson, maladie de Huntington, démence vasculaire, maladie à corps de Lewy, démence fronto-temporale, syndrome de Tourette, hyperprolactinémie, obésité, et trouble de stress post-traumatique (TSPT).

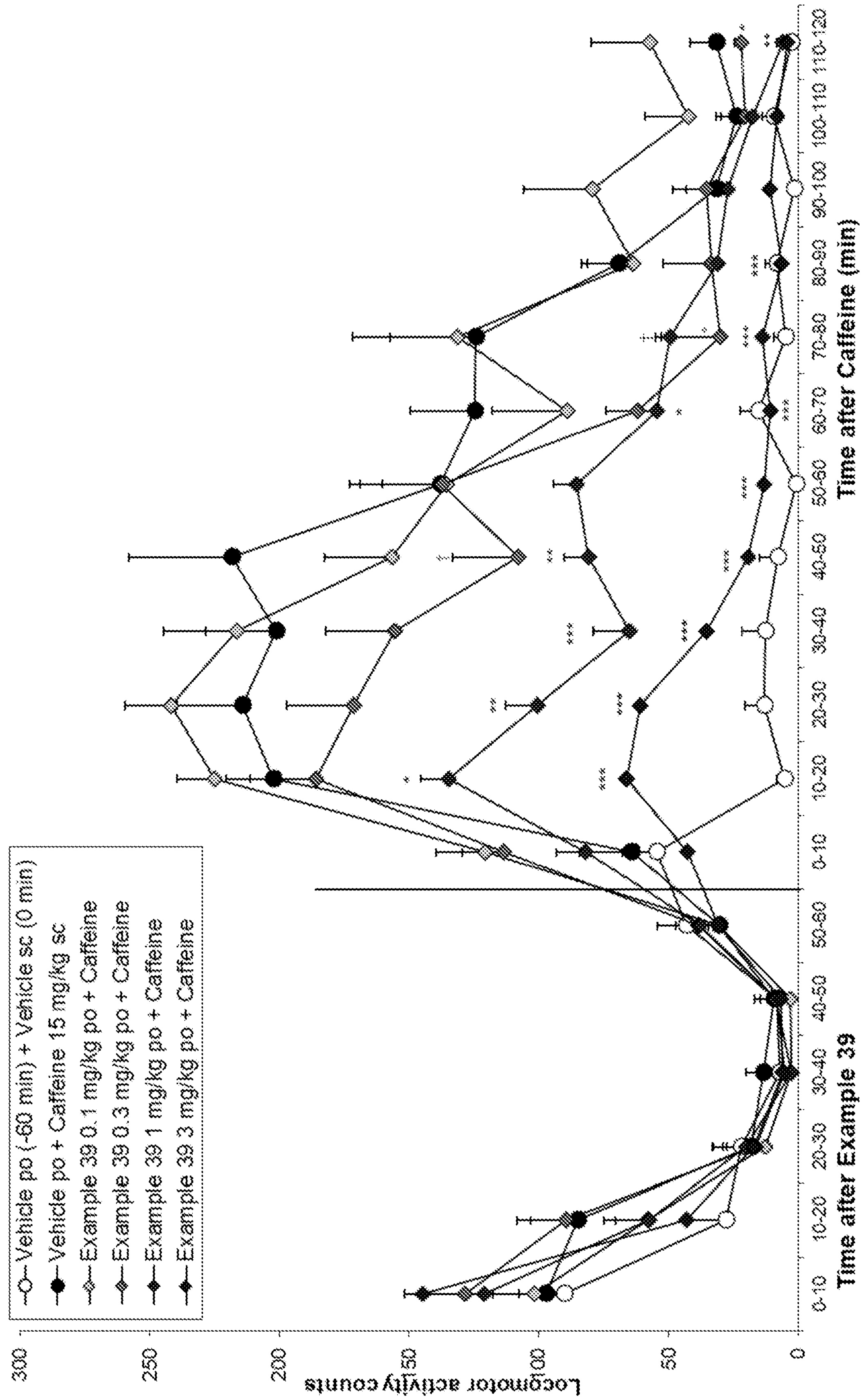
Vehicle po (-60 min) + Vehicle sc (0 min)
Vehicle po + Caffeine 15 mg/kg sc
Example 39 0.1 mg/kg po + Caffeine
Example 39 0.3 mg/kg po + Caffeine
Example 39 1 mg/kg po + Caffeine
Example 39 3 mg/kg po + Caffeine
Locomotor activity counts
Time after Example 39
Time after Caffeine (min)

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016176571 A, Xiong
- WO 2021090030 A
- GB 2021050638 W
- WO 2021181122 A1


### Non-patent literature cited in the description


- **KOMATSU et al.** *PLoS One*, 2014, vol. 9, e90134
- **RAUTIO, J. et al.** *Nat. Rev. Drug Discov.*, 2018, vol. 17, 559-587
- *Top. Curr. Chem*, 2015, vol. 360, 115-160
- **BRYN et al.** , Solid-State Chemistry of Drugs. SSCI, Inc of West Lafayette, 1999
- Remington's Pharmaceutical Sciences. Mack Publishing Company
- *Curr. Pharm. Des.*, 2008, vol. 14, 1468
- *Pharmacol. Biochem. Behav.*, 1994, vol. 47, 89
- *Naunyn-Schmiedeberg's Arch. Pharmacol.*, 2016, vol. 389, 11